(19) Europäisches Patentamt
European Patent Office
Office européen des brevets

(11) EP 0 820 989 A1

(12) **EUROPEAN PATENT APPLICATION**
published in accordance with Art. 158(3) EPC

(43) Date of publication:
**28.01.1998 Bulletin 1998/05**

(21) Application number: **96904295.1**

(22) Date of filing: **01.03.1996**

(51) Int. Cl.[6]: **C07D 233/58**, C07D 403/06, A61K 31/415

(86) International application number:
**PCT/JP96/00490**

(87) International publication number:
**WO 96/26927 (06.09.1996 Gazette 1996/40)**

(84) Designated Contracting States:
**AT BE CH DE DK ES FI FR GB GR IE IT LI LU NL PT SE**

(30) Priority: **01.03.1995 JP 42067/95**

(71) Applicant:
**YAMANOUCHI PHARMACEUTICAL CO., LTD.
Tokyo 103 (JP)**

(72) Inventors:
- **YODEN, Toru**
  **Tsukuba-shi, Ibaraki 305 (JP)**
- **OKADA, Minoru**
  **Toride-shi, Ibaraki 302 (JP)**
- **KINOYAMA, Isao**
  **Ushiku-shi, Ibaraki 300-12 (JP)**
- **ISHIHARA, Tsukasa**
  **Tsukuba-shi, Ibaraki 305 (JP)**
- **SAKUDA, Syuichi**
  **Tsukuba-shi, Ibaraki 305 (JP)**
- **IDEYAMA, Yukitaka**
  **Tsukuba-shi, Ibaraki 305 (JP)**
- **KUDOH, Masafumi**
  **Kitasouma-gun, Ibaraki 305-01 (JP)**

(74) Representative:
**Geering, Keith Edwin
REDDIE & GROSE
16 Theobalds Road
London WC1X 8PL (GB)**

(54) **IMIDAZOLE DERIVATIVES AND MEDICINAL COMPOSITION THEREOF**

(57) An imidazole derivative represented by the following general formula (I), a salt thereof, a hydrate thereof or a solvate thereof.

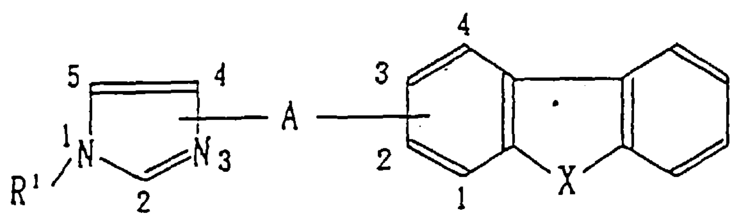

(I)

(Symbols in the formula have the following meanings.

A: a lower alkylene group unsubstituted or substituted with a hydroxyl group, an aryl group, a lower alkylidene group or an oxo group (=O),
X: a methylene group or a group represented by a formula $-NR^2-$,
$R^1$: a hydrogen atom, a lower alkyl group or an aralkyl group,
$R^2$: a hydrogen atom or a lower alkyl group). The compounds have a steroid 17-20 lyase inhibiting activity and is useful in treating and preventing diseases such as prostate cancer, benign prostatic hyperplasia, breast cancer, mastopathy, hysteromyoma, endometriosis and the like.

EP 0 820 989 A1

## Description

### TECHNICAL FIELD

This invention relates to a novel imidazole derivative, particularly a novel imidazole derivative having carbazole or fluorene or a salt thereof, which shows a steroid 17-20 lyase inhibiting activity and is useful as a medicine.

### BACKGROUND ART

It is known that an enzyme, called steroid 17-20 lyase, plays a role in the production of androgen from cholesterol in the body at the final stage of its biosynthetic pathway. Steroid 17-20 lyase uses 17α-hydroxypregnenolone and 17α-hydroxyprogesterone as the substrates, which are synthesized from cholesterol and have a carbon substituent at the 17β-position, and cleaves the bonding between the 17-position carbon and the 20-position carbon of the carbon substituent, thereby forming dehydroepiandrosterone and androstenedione, respectively. In consequence, the production of androgen as well as estrogen, which is synthesized from androgen as the substrate, could be decreased by inhibiting the enzyme activity of steroid 17-20 lyase, which makes it possible to prevent and treat various diseases in which androgen and/or estrogen take part as aggravating factors. Examples of such diseases in which androgen and estrogen take part as aggravating factors include prostate cancer, benign prostatic hyperplasia, virilism, hirsutism, breast cancer, mastopathy, hysteromyoma, endometriosis and the like.

On the other hand, it is well established that reduction of the serum androgen level is useful in treating various diseases such as prostate cancer and the like. Orchiectomy, an LH-RH agonist or an androgen antagonist is used in the clinical practice. However, orchiectomy is mentally unacceptable and the LH-RH agonist can block androgen from the testis but not from other organs, and exhibits a transient flare phenomenon due to its agonist action. In the case of the androgen antagonist, it has been known recently that its effect is attenuated by the mutation of androgen receptors. In consequence, it has been proposed to block effects of androgen on receptors (total androgen blocked), and attempts have been made to use LH-RH agonist and androgen antagonist in combination.

A compound which inhibits steroid 17-20 lyase is considered to be a drug that can perform total androgen blocked by strongly inhibiting androgen action due to its function and therefore is expected to be a useful drug for the treatment of prostate cancer and the like. In addition, being capable of reducing estrogen, a steroid 17-20 lyase inhibitor is expected to be more effective therapeutic agent than a therapeutic agent which can only block androgen action in the treatment of benign prostatic hyperplasia and is expected to be a drug with less side effects.

As steroid 17-20 lyase inhibitors, steroidal and non-steroidal compounds have been synthesized. As an example of the non-steroidal inhibitor of steroid 17-20 lyase, a (1H-imidazol-1-ylmethyl)-substituted benzimidazole derivative disclosed in an unexamined published Japanese patent application (*Kokai*) No. 64-85975 is known, which is a compound in which a substituted benzimidazolyl group and an imidazolyl group are linked by a methine carbon or a methylene carbon.

Also, International Publication WO 94/27989 discloses that a carbazole derivative substituted with pyridin-3-ylmethyl group, pyridin-4-ylmethyl group or [1,2,4]triazol-1-ylmethyl group has a steroid 17-20 lyase inhibiting activity. However, the inhibitory activity of these compounds toward steroid 17-20 lyase is still insufficient.

In addition, the inventors of the present invention have reported that an imidazolylalkylamine derivative represented by the following general formula (A)

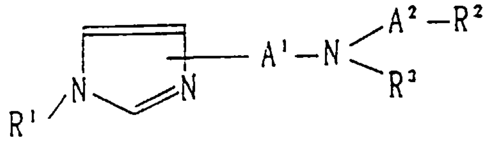

(A)

as disclosed in the previously filed International Publication WO 95/04723 has a steroid 17-20 lyase inhibiting activity. The $R^2$ of said general formula (A) is "a substituted or unsubstituted phenyl group, a substituted or unsubstituted bicyclic or tricyclic hydrocarbon ring group having condensed benzene ring, or a substituted or unsubstituted bicyclic or tricyclic condensed heterocyclic ring group having a heterocyclic ring containing an oxygen atom(s) and/or a sulfur atom(s) and/or a nitrogen atom(s) as the hetero atom and a condensed benzene ring", and carbazolyl group and fluorenyl group are disclosed as its illustrative examples. However, though these compounds have excellent activity to inhibit steroid 17-20 lyase *in vitro*, its pharmacological effect *in vivo* is still insufficient.

As described above, various studies have been made, but development of an excellent steroid 17-20 lyase inhibitor is still an important subject in the field of medical treatment.

DISCLOSURE OF THE INVENTION

The inventors of the present invention conducted extensive studies and, as the result, found that the steroid 17-20 lyase inhibiting activity of an imidazole derivative represented by the following general formula (I) in which an imidazole ring is linked with a carbazole ring or a fluorene ring via a lower alkylene group, or a salt thereof, is excellent not only *in vitro* but also *in vivo*, thus resulting in the accomplishment of the present invention.

Accordingly, the present invention is an imidazole derivative represented by the following general formula (I), a salt thereof, a hydrate thereof or a solvate thereof

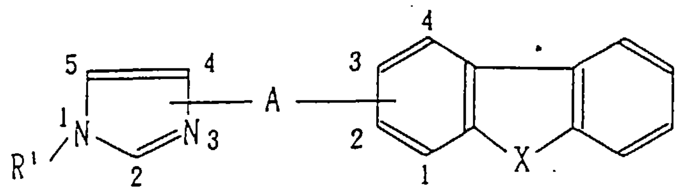

(I)

(symbols in the formula have the following meanings;

A: a lower alkylene group unsubstituted or substituted with a hydroxyl group, an aryl group, a lower alkylidene group or an oxo group (=O),
X: a methylene group or a group represented by a formula $-NR^2-$,
$R^1$: a hydrogen atom, a lower alkyl group or an aralkyl group, and
$R^2$: a hydrogen atom or a lower alkyl group).

Among the compounds (I) of the present invention, a preferable compound is a compound wherein it is characterized in that A is linked at the 4-position or 5-position of the imidazole ring, a salt thereof, a hydrate thereof or a solvate thereof;

more preferably an imidazole derivative wherein it is characterized in that A is linked at the 2-position or 3-position of the ring

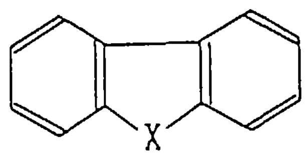

,

a salt thereof, a hydrate thereof or a solvate thereof;
an imidazole derivative wherein A is a lower alkylene group unsubstituted or substituted with an aryl group or a lower alkylidene group, a salt thereof, a hydrate thereof or a solvate thereof;
particularly preferably an imidazole derivative wherein A is a lower alkylene group unsubstituted or substituted with a lower alkylidene group, a salt thereof, a hydrate thereof or a solvate thereof;
an imidazole derivative wherein X is a group represented by the formula $NR^2$;
most preferably 2-[1-(1H-imidazol-4-yl)ethyl]-9H-carbazole, a salt thereof, a hydrate thereof or a solvate thereof;
3-[1-(1H-imidazol-4-yl)ethyl]-9H-carbazole, a salt thereof, a hydrate thereof or a solvate thereof;
2-(1H-imidazol-4-ylmethyl)-9H-carbazole, a salt thereof, a hydrate thereof or a solvate thereof; and
3-(1H-imidazol-4-ylmethyl)-9H-carbazole, a salt thereof, a hydrate thereof or a solvate thereof.

Also, the pharmaceutical composition which contains the compound of the present invention, as another object of the present invention, is a steroid 17-20 lyase inhibitor which comprises a compound represented by the aforementioned general formula (I) or a pharmaceutically acceptable salt thereof as its active ingredient;

particularly, a steroid 17-20 lyase inhibitor which is an agent for prevention or treatment of diseases caused by androgen and/or estrogen;
illustratively a steroid 17-20 lyase inhibitor which is an agent for the prevention or treatment of prostate cancer,

benign prostatic hyperplasia, virilism, hirsutism, breast cancer, mastopathy, hysteromyoma and endometriosis.

The following describes the present invention in detail.

Unless otherwise noted, the term "lower" as used herein in the definition of the general formula means a straight or branched carbon chain having 1 to 6 carbon atoms.

Illustrative examples of the "lower alkyl group" include methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl and the like. Among these groups, alkyl groups having 1 to 4 carbon atoms such as methyl, ethyl, propyl, isopropyl, butyl and the like are preferable.

Illustrative examples of the "lower alkylene group" include methylene, ethylene, methylmethylene, trimethylene, 1-methylethylene, 2-methylethylene, ethylmethylene, tetramethylene, 1-methyltrimethylene, 2-methyltrimethylene, 3-methyltrimethylene, 1-ethylethylene, 2-ethylethylene, 1,2-dimethylethylene, propylmethylene, isopropylmethylene, pentamethylene, 1-methyltetramethylene, 2-methyltetramethylene, 3-methyltetramethylene, 4-methyltetramethylene, 1-ethyltrimethylene, 2-ethyltrimethylene, 3-ethyltrimethylene, 1,1-dimethyltrimethylene, 2,2-dimethyltrimethylene, 3,3-dimethyltrimethylene, hexamethylene, 1-methylpentamethylene, 2-methylpentamethylene, 3-methylpentamethylene, 4-methylpentamethylene, 5-methylpentamethylene, 1,1-dimethyltetramethylene, 4,4-dimethyltetramethylene and the like.

Among these groups, alkylene groups having 1 to 4 carbon atoms are preferred, and methylene, methylmethylene, ethylmethylene, propylmethylene, isopropylmethylene and the like are more preferred.

The "lower alkylidene group" is a straight or branched alkylidene group having 2 to 6 carbon atoms, and its illustrative examples include methylidene ($CH_2$=), ethylidene ($CH_3$—CH=), propylidene ($CH_3$—$CH_2$—CH=), isopropylidene

( 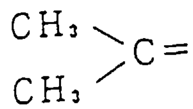 ),

butylidene ($CH_3$—$CH_2$—$CH_2$—CH=), methylpropylidene, pentylidene, methylbutylidene, ethylpropylidene, hexylidene, methylpentylidene, ethylbutylidene, propylpropyiidene and the like, of which methylidene, ethylidene or isopropylidene is preferred. The lower alkylene group substituted with a lower alkylidene group is a group in which two hydrogen atoms linked to an optional carbon atom of a lower alkylene group are substituted with the double bond of a lower alkylidene group, and its preferred examples include vinylidene

( 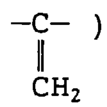 ),

exoisopropylidenemethylene

( 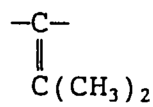 ),

exoethylidenemethylene

( 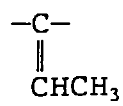 ),

exomethyleneethylene

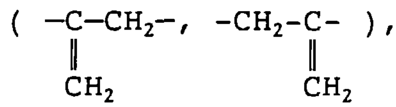

and exoisopropylideneethylene

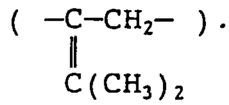

The aforementioned "lower alkylene group" may be substituted with hydroxyl group, an aryl group or an oxo group (=O) at an optional position.

In this case, the term "aryl group" means a carbon ring aryl, and its illustrative examples include phenyl, biphenyl, naphthyl and the like, of which phenyl is preferred. In consequence, illustrative examples of the lower alkylene group which may be substituted with hydroxyl group, an aryl group or an oxo group include hydroxymethylene

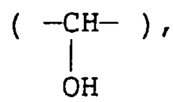

(hydroxy)(methyl)methylene

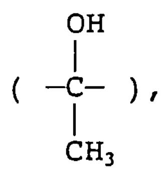

hydroxyethylene

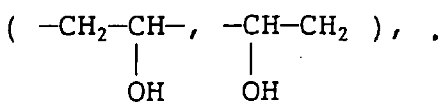

hydroxytrimethylene

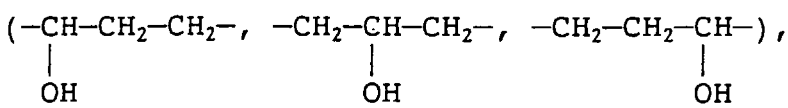

phenylmethylene

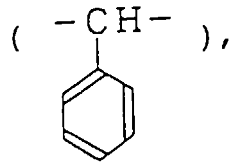

benzylmethylene

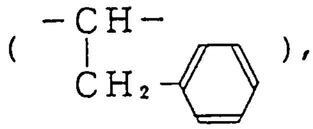

phenylethylene

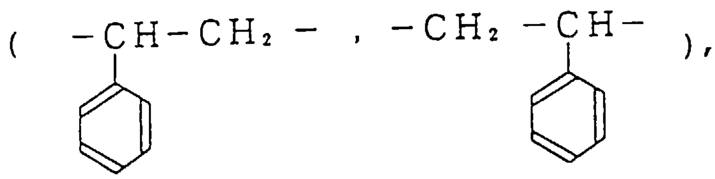

benzylethylene

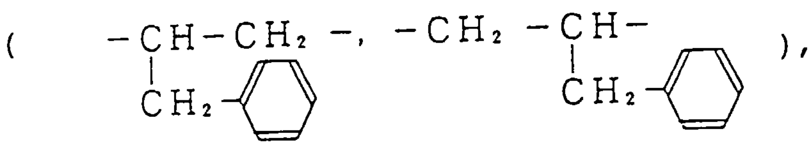

phenyltrimethylene

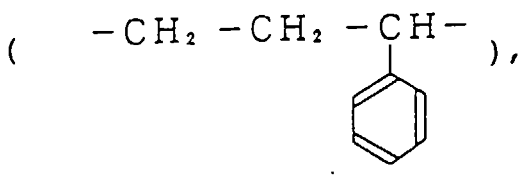

naphthylmethylene

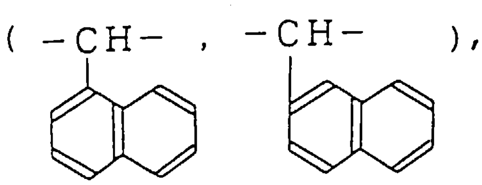

carbonyl

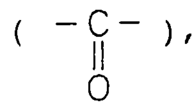

carbonylmethylene

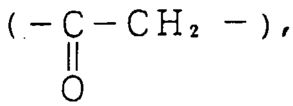

carbonylethylene

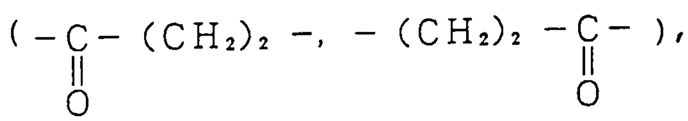

and the like.

As the substituent for the "lower alkylene group", a lower alkylidene group having 1 to 3 carbon atoms, phenyl group, or the like is preferable.

The "aralkyl group" means a group in which the aforementioned aryl group is substituted at an optional position of the aforementioned lower alkyl group, and its illustrative examples include benzyl, phenetyl, naphthylmethyl and the like.

The compound of the present invention can form a salt with an inorganic acid or an organic acid in some cases, and these salts also have the steroid 17-20 lyase inhibiting activity as well as the free base. Examples of preferable salts include salts with inorganic acid such as of hydrochloric acid, hydrobromic acid, hydroiodic acid, sulfuric acid, nitric acid, phosphoric acid and the like and with organic acid such as of formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid and the like.

Depending on the kind of substituents, it may also form a salt with a pharmaceutically acceptable alkali metal or alkaline earth metal (for example, sodium, potassium or magnesium, or calcium) or a salt with ammonia or an organic amine such as triethylamine or the like.

The compound of the present invention may have an asymmetric carbon atom depending on the kind of the aforementioned group A or depending on substituents, and such a compound exists in optical isomer forms based on the asymmetric carbon atom, or further in diastereoisomer forms when it has two or more asymmetric carbon atoms. In addition, it may also exist in geometrical isomer forms (cis form and trans form) or tautomer forms based on the double bond. Isolated forms of these various isomers and mixtures thereof are all included in the present invention.

Also, the compound of the present invention may exist in the form of various hydrates, various solvates such as with methanol, ethanol and the like, tautomers, polymorphic forms and the like, and isolated forms of these compounds and mixtures thereof are all included in the present invention.

The compound of the present invention can be produced by employing various synthetic methods.

Its typical production methods are exemplified in the following.

In this connection, when X in the compound (I) of the present invention prevents the reactions described below, the reactions can be carried out using appropriate protecting groups. For example, when X is -NH-, it is converted into -$NP^1$- using a protecting group $P^1$. The group $P^1$ is a conventional amino-protecting group, and its illustrative examples include a benzyl-type protecting group such as benzyl, benzhydryl, trityl, 4-methoxybenzyl or the like, an acyl group such as formyl, acetyl, propionyl or the like, an aralkyloxycarbonyl group such as benzyloxycarbonyl or the like, a lower alkoxycarbonyl group such as tert-butoxycarbonyl or the like, a sulfonyl group such as tosyl, benzenesulfonyl, methanesulfonyl or the like, an aminoacetal-type protecting group such as methoxymethyl, tetrahydropyranyl or the like, and a trialkylsilyl group such as trimethylsilyl, tert-butyldimethylsilyl or the like.

Method 1

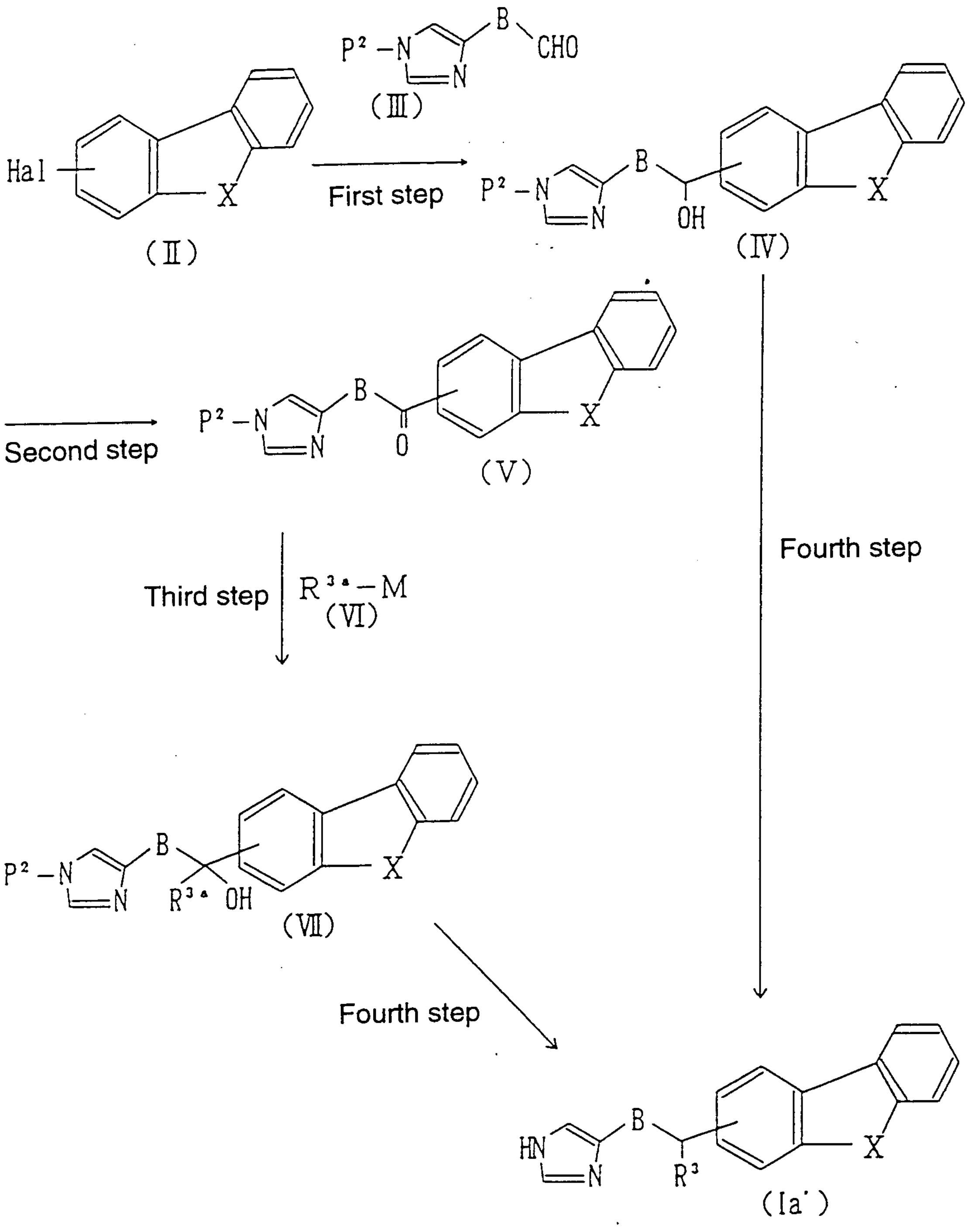


(Symbols in the formula are as follows;

Hal: a halogen atom,
B: a single bond, or an alkylene group which has 1 to 5 carbon atoms and may be substituted with an aryl group,
$P^2$: a benzyl-type protecting group such as trityl or the like,
$R^{3a}$: a lower alkyl group or an aryl group,

$R^3$: a hydrogen atom, a lower alkyl group or an aryl group, and

$R^{3a}$-M: an organometallic reagent such as an alkyl lithium, an aryl lithium, a Grignard's reagent or the like

(M is lithium, -Mg-Hal or the like)

the same shall apply hereinafter.)

The halogen atom means fluorine atom, chlorine atom, bromine atom or iodine atom.

This production method is carried out in the following manner.

First step: This is a step in which a secondary alcohol (IV) is obtained by allowing a halogen compound (II) to react with a metal reagent (an alkyl lithium such as n-butyl lithium or the like or metallic magnesium or the like) in an organic solvent such as tetrahydrofuran (THF), dioxane, diethyl ether, dimethoxyethane or the like, and then allowing the resulting organometallic reagent to react with its reaction-corresponding amount, preferably 1 to 2 equivalents, of an aldehyde compound (III) at low temperature, preferably at -100 to 0°C.

Second step: This is a step in which a ketone compound (V) is obtained from the secondary alcohol (IV) by a conventional oxidation reaction with an oxidizing agent (manganese dioxide, chromic acid or the like) in an organic solvent such as dichloromethane, dichloroethane, chloroform, THF, dioxane, benzene or the like.

Third step: This is a step in which a tertiary alcohol (VII) is obtained by allowing the ketone compound (V) to react with its reaction-corresponding amount, 1 to 2 equivalents, of an organometallic reagent (VI) (Grignard's reagent, alkyl lithium reagent or the like) in an organic solvent such as THF, dioxane, diethyl ether, dimethoxyethane or the like at 0°C to room temperature.

The above second and third steps are carried out when $R^3$ is a lower alkyl group or an aryl group.

Fourth step: This is a step in which an imidazole compound (Ia') is obtained by allowing the secondary alcohol (IV) or the tertiary alcohol (VII) to undergo the reaction in the presence of 10 to 100% by weight of a catalyst (10% palladium-carbon, palladium, palladium hydroxide, palladium oxide, platinum, platinum oxide or the like) in an organic solvent such as methanol, ethanol, acetic acid or the like under hydrogen atmosphere at room temperature to reflux temperature.

In this connection, depending on the kind of the protecting group $P^2$ (e.g., trityl group), a deprotection reaction may be carried out by treating it under an acidic condition after completion of the reaction of the first step (when $R^3$ is a hydrogen atom) or the third step (when $R^3$ is a lower alkyl group or an aryl group), thereby preparing a secondary alcohol (IVa) or a tertiary alcohol (VIIa) shown below, from which the compound (Ia') is obtained by the fourth step.

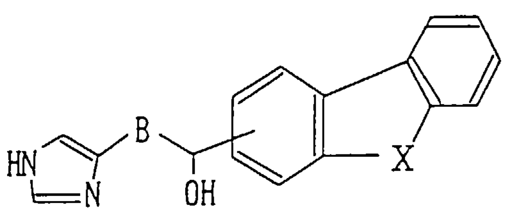

(IVa)

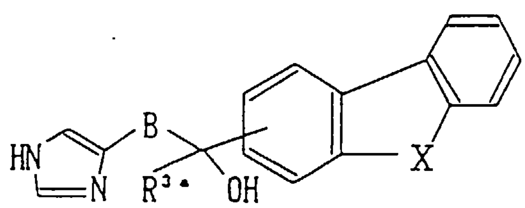

(VIIa)

Also, the tertiary alcohol (VII) can be obtained without using the second step and third step, by the use of a ketone compound (the following formula IIIa) instead of the aldehyde compound (III) in the first step. In the same manner, a substituted imidazole (Ib) can be obtained without using the Method 2, by the use of an aldehyde compound (IIIb) instead of the aldehyde compound (III) in the first step.

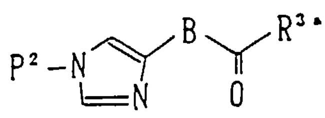

(IIIa),

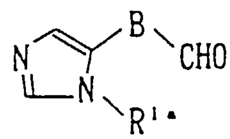

(IIIb)

(In the above formulae, $R^{1a}$ represents a lower alkyl group or an aralkyl group; the same shall apply hereinafter.)

Method 2

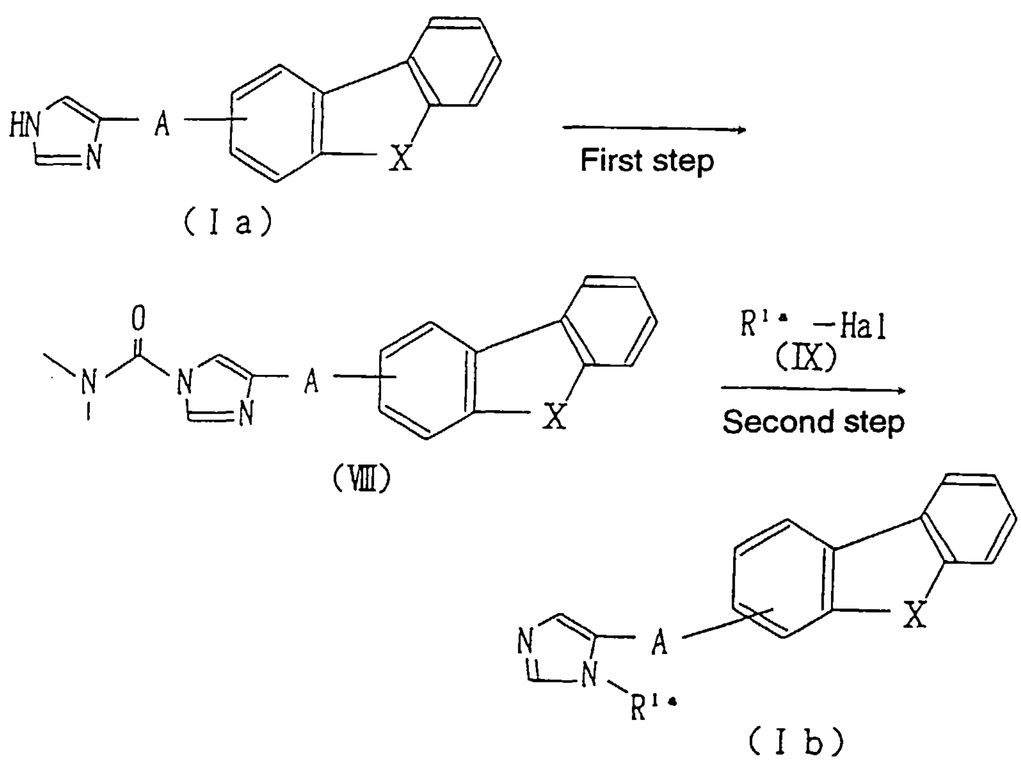


In this production method, a substituted imidazole (Ib) is obtained.

First step: This is a step in which a carbamoyl compound (VIII) is obtained by allowing the imidazole compound (Ia) to react with its reaction-corresponding amount, 1 to 5 equivalents, of dimethylcarbamoyl chloride in an organic solvent such as acetonitrile, THF, dimethylformamide (DMF) or the like at room temperature to reflux temperature.
Second step: This is a step in which the substituted imidazole compound (Ib) is obtained by allowing the carbamoyl compound (VIII) to react with reaction-corresponding amount or excess amount of an alkyl halide or aralkyl halide (IX) in an organic solvent such as acetonitrile, THF, DMF or the like at room temperature to reflux temperature to give an onium compound, and then allowing the resulting onium compound to react with ammonia.

Method 3

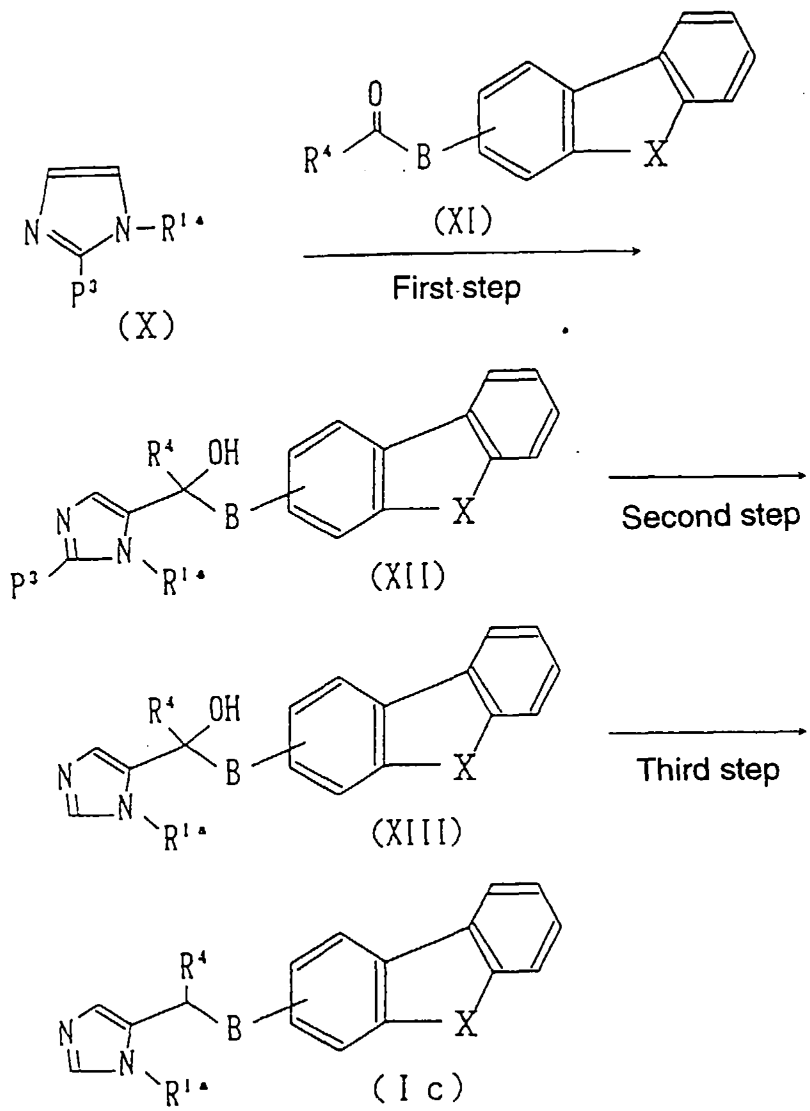


(X) (XI) (XII) (XIII) (I c)

(Symbols in the formula are as follows;

$P^3$: a protecting group such as an alkylthio group, an arylthio group or the like, and
$R^4$: a hydrogen atom, a lower alkyl group or an aryl group;

the same shall apply hereinafter.)

In this production method, a substituted imidazole (Ic) is obtained.

First step: This is a step in which a protected imidazolyl-substituted alcohol (XII) is obtained by allowing an imidazole (X) protected with an alkylthio group or an arylthio group to react with a base (an alkyl lithium such as n-butyl lithium or the like or a lithium amide reagent such as lithium diisopropylamide or the like) in an organic solvent such as THF, dioxane, diethyl ether, dimethoxyethane or the like, and then allowing the resulting organometallic reagent to react with its reaction corresponding amount, 1 to 2 equivalents, of an aldehyde or ketone (XI) at low temperature, preferably at -100 to 0°C.

Second step: This is a step in which an imidazolyl-substituted alcohol (XIII) is obtained by removing the protecting

group of the imidazole ring of the compound (XII) by allowing it to undergo the reaction in an organic solvent such as methanol, ethanol or the like in the presence of Raney nickel or the like at room temperature to reflux temperature.

Third step: This is a step in which a compound (Ic) of the present invention is obtained by allowing the imidazolyl-substituted alcohol (XIII) to undergo the reaction in the presence of 10 to 100% by weight of a catalyst (10% palladium-carbon, palladium, palladium hydroxide, palladium oxide, platinum, platinum oxide or the like) in an organic solvent such as methanol, ethanol, acetic acid or the like under hydrogen atmosphere at room temperature to reflux temperature.

(Alternative method)

The compound (Ic) of the present invention can be obtained by adding an acid such as trifluoroacetic acid, trifluoroborane or the like to the imidazolyl-substituted alcohol (XIII) in an organic solvent such as dichloromethane, dichloroethane, chloroform, THF, dioxane, benzene, trifluoroacetic acid or the like, and carrying out the reaction at 0°C to reflux temperature in the presence of the reaction-corresponding amount or excess amount of a reducing agent such as a trialkylsilane or the like.

Method 4

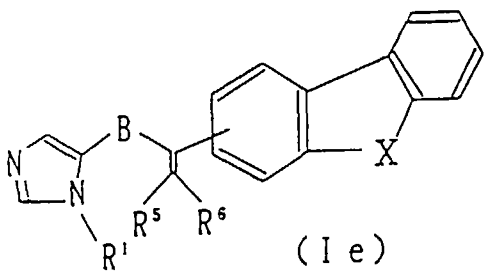

(I e)

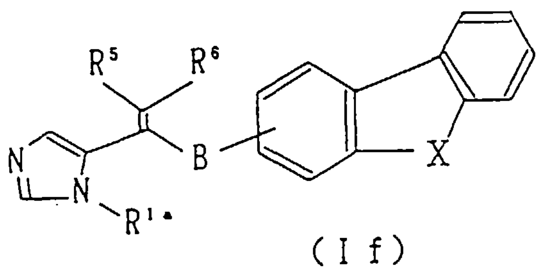

(I f)

(In the above formulae, $R^5$ and $R^6$ may be the same or different from each other, and each represents a hydrogen atom or a lower alkyl group.)

This is a method to prepare a compound (Ie) or (If) of the present invention which has a lower alkylidene group.

The compound (Ie) or (If) can be prepared by carrying out dehydration of the aforementioned tertiary alcohol (VII) or (XIII) of the Method 1 or 3, under an acidic condition with an acid such as hydrochloric acid, acetic acid, trifluoroacetic acid, p-toluenesulfonic acid or the like in an organic solvent such as methanol, ethanol, THF, dioxane, dichloromethane, dichloroethane, chloroform, benzene, acetic acid, trifluoroacetic acid or the like, and then carrying out the steps shown in the respective production methods as occasion demands.

Method 5

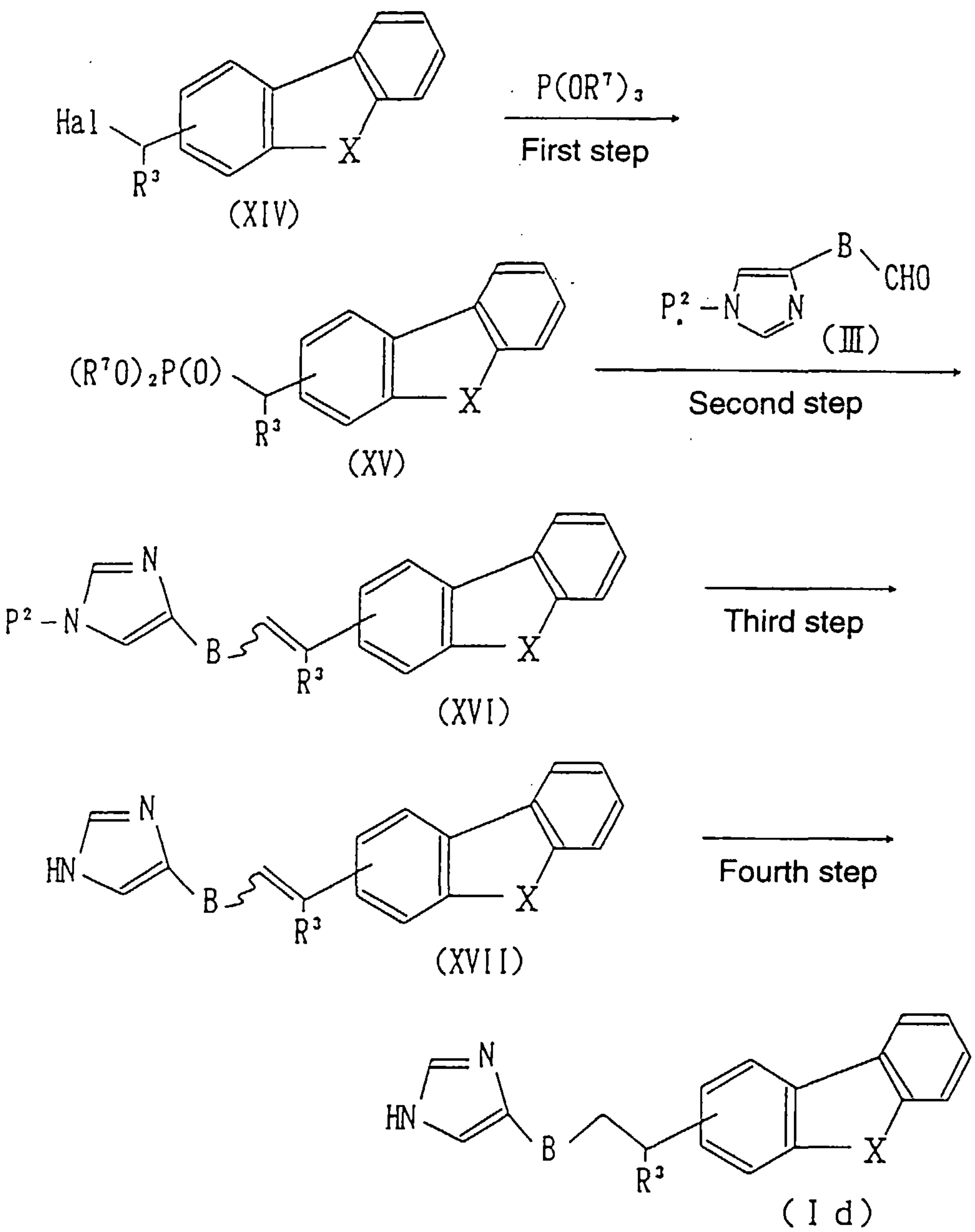


(XIV) (XV) (XVI) (XVII) (I d)

(In the above formulae, $R^7$ is a lower alkyl group.)

First step: This is a step in which a phosphonate compound (XV) is obtained by allowing a halomethyl compound (XIV) to react with its reaction-corresponding amount of a trialkyl phosphite in an organic solvent such as THF, dioxane, benzene, DMF or the like at room temperature to reflux temperature.

Second step: This is a step in which an olefinic compound (XVI) (includes two geometrical isomers) is obtained by allowing the phosphonate (XV) to react with its reaction corresponding amount, 1 to 2 equivalents, of an aldehyde compound (III) at 0°C to reflux temperature in an organic solvent such as THF, dioxane, dimethoxyethane, diethyl ether, benzene or the like in the presence of a base (sodium hydride, potassium hydride, potassium tert-butoxide, potassium bistrimethylsilylamide, an alkyl lithium such as n-butyl lithium or the like, or a lithium amide such as lithium diisopropylamide or the like, etc.).

Third step: This is a step in which deprotection of the imidazole ring is carried out by allowing the olefinic compound (XVI) obtained in the second step to undergo the reaction under an acidic (hydrochloric acid, acetic acid, trifluoroacetic acid, p-toluenesulfonic acid or the like) condition in an organic solvent such as methanol, ethanol, THF, dioxane, acetic acid, trifluoroacetic acid or the like at room temperature to reflux temperature.

Fourth step: This step is carried out by a conventional catalytic hydrogenation method. In this step, a compound (Id) of the present invention is obtained by reducing the olefinic compound (XVII) obtained in the third step in the presence of a catalyst (palladium, palladium hydroxide, palladium oxide, platinum, platinum oxide or the like) in an organic solvent such as methanol, ethanol, ethyl acetate, THF, dioxane or the like under hydrogen atmosphere.

When the protecting group $P^2$ can be removed by catalytic hydrogenation, the compound (Id) can be obtained by the fourth step directly from the compound (XVI) without employing the third step.

Method 6

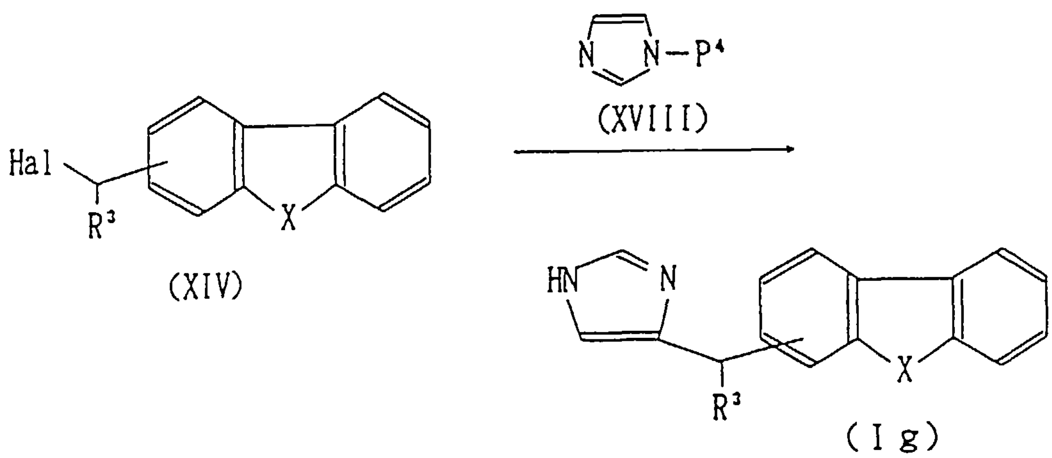

(XVIII)

(XIV)

(Ig)

(In the above formulae, $P^4$ represents a protecting group such as trimethylsilyl or the like.)

This production method is an alternative method for the production of the compound (Ig) of the present invention which has a methylene group.

The compound (Ig) can be prepared by allowing a halomethyl compound (XIV) to react with an imidazole (XVIII) protected with a protecting group such as a trimethylsilyl group or the like in an organic solvent such as chloroform or the like in the presence of a Lewis acid catalyst such as titanium tetraoxide or the like, with cooling or at room temperature.

In any of these production methods, elimination of protecting groups is carried out in the conventional manner. For example, it may be effected by an oxidation reaction, a reduction reaction or a hydrolysis reaction under an acidic or basic condition.

The thus prepared compounds of the present invention are isolated and purified as a free compound or as a salt thereof, a hydrate thereof, various types of solvate thereof or in a polymorphic form. A pharmaceutically acceptable salt of the compound (I) of the present invention can be produced by applying the compound to a usually used salt formation reaction.

Isolation and purification are carried out by applying usual chemical procedures such as extraction, fractional crystallization, various types of fractional chromatography and the like.

In addition, optical isomers can be separated as a stereochemically pure isomer by selecting an appropriate starting material compound or by employing a racemic resolution method of racemic compounds (such as a method in which a diastereomer salt is formed with a general optically active acid and then subjected to optical resolution).

## INDUSTRIAL APPLICABILITY

The compound of the present invention has a function to inhibit the activity of steroid 17-20 lyase which is an enzyme that plays a role in the formation of androgen from cholesterol in the body. In consequence, because of its

action to inhibit synthesis of androgen and of estrogen which is synthesized from androgen as the substrate, the compound of the present invention is useful as an agent for the prevention and treatment of various diseases in which androgen and estrogen take part as aggravating factors, such as prostate cancer, benign prostatic hyperplasia, virilism, hirsutism, breast cancer, mastopathy, hysteromyoma, endometriosis and the like.

The usefulness of the compound of the present invention has been confirmed by the following tests.

(1) Measurement of inhibition of rat steroid 17-20 lyase activity [*in vitro*]

This was carried out in accordance with the method described in *J. Steroid Biochem.*, Vol.33, No.6, 1191-1195 (1989).

Testes were excised from ten-week-old male Wistar rats, homogenized and then centrifuged to give microsomes. The microsomal protein (50 μg), 1 μM of [1,2-$^3$H]-17α-hydroxyprogesterone (5.55 × $10^5$ dpm) and a test compound were dissolved in 100 μl of 50 mM phosphate buffer (pH 7.4), NADPH solution was added, and then the mixture was incubated at 37°C for 60 minutes. Then, 400 μl of a mixed solution of methanol and tetrahydrofuran (2:3) was added, and the mixture was subjected to centrifugation, and then radioactivities of the substrate and the formed product (androstenedione and testosterone) in the resulting supernatant were measured by a high performance liquid chromatography (HPLC) equipped with a radioisotope detector to examine the activity of the test compound to inhibit steroid 17-20 lyase. The results are shown in Table 1.

Table 1

| Test compounds | Inhibition of steroid 17-20 lyase activity $IC_{50}$ |
|---|---|
| Example 1e | 3.9 nM |
| Example 13 | 2.5 nM |
| Control compound 1) | 12.7 nM |

1)

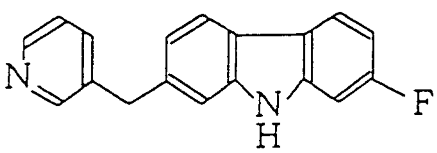

(WO 94/27989, Example 2)

It was confirmed from the above results that the compounds of the present invention have excellent effects in comparison with the control compound.

(2) Measurement of inhibition of rat testosterone synthesis [*in vivo*]

This was carried out in accordance with the method described in *J. Steroid Biochem.*, Vol.32, No.6, 781-788 (1989).

Each test compound was orally administered to ten-week-old male Wistar rats. After a predetermined period of the drug administration, each animal was decapitated to collect blood, and testosterone concentration in the thus prepared serum sample was measured by a radioisotope assay to calculate inhibition of testosterone synthesis. As the result, the compound of the present invention showed a strong activity to inhibit testosterone synthesis.

(3) Reduction in weight of prostate in rats

Each test compound was orally administered every day to ten-week-old male Wistar rats, and weight of the prostate was measured two weeks thereafter. As the result, the compound of the present invention showed a strong action to reduce weight of the prostate.

As the results of these pharmacological tests, it was confirmed that the compound of the present invention has excellent activity to inhibit steroid 17-20 lyase both *in vitro* and *in vivo*. In addition, the compound of the present invention is also excellent in terms of duration of action and enzyme specificity.

The pharmaceutical composition which contains one or two or more of the compounds of the present invention represented by the general formula (I) and pharmaceutically acceptable salts, hydrates and the like thereof as the active ingredient is prepared into tablets, powders, fine granules, granules, capsules, pills, solutions, injections, suppositories and the like using generally used pharmaceutical carriers, excipients and other additives, and are administered orally or parenterally.

The dose is optionally decided by taking into consideration symptoms, age, sex, weight and the like of each patient to be treated, but the drug is orally administered within the range of usually from 0.1 to 100 mg, preferably from 0.1 to 10 mg, per day per adult, by dividing the daily dose into one to several doses per day, or parenterally administered within the range of from 0.1 to 100 mg per day per adult, by dividing the daily dose into one to several doses per day, or within the range of from 1 to 24 hours a day by continuous intravenous infusion. As a matter of course, since the dose varies under various conditions as described in the foregoing, a smaller dose than the above range may be sufficient in some cases.

The solid composition for use in the oral administration according to the present invention include tablets, powders, granules and the like. In such a solid composition, one or more active substances are mixed with at least one inert diluent such as lactose, mannitol, glucose, hydroxypropylcellulose, microcrystalline cellulose, starch, polyvinyl pyrrolidone, aluminum magnesium silicate. In the usual way, the composition may contain other additives than the inert diluent, such as a lubricant (e.g., magnesium stearate or the like), a disintegrating agent (e.g., calcium cellulose glycolate or the like), a stabilizing agent (e.g., lactose or the like) and a solubilization assisting agent (e.g., glutamic acid, aspartic acid or the like). If necessary, tablets or pills may be coated with a film of a gastric or enteric substance such as sucrose, gelatin, hydroxypropylcellulose, hydroxypropylmethylcellulose phthalate or the like.

The liquid composition for oral administration includes pharmaceutically acceptable emulsions, solutions, suspensions, syrups, elixirs and the like and contains a generally used inert diluent such as purified water or ethanol. In addition to the inert diluent, this composition may also contain auxiliary agents such as a moistening agent, a suspending agent and the like, as well as sweeteners, flavors, aromas and antiseptics.

The injections for parenteral administration includes aseptic aqueous or non-aqueous solutions, suspensions and emulsions. Examples of the aqueous solutions and suspensions include distilled water for injection use and physiological saline. Examples of the non-aqueous solutions and suspensions include propylene glycol, polyethylene glycol, plant oils such as olive oil or the like, alcohols such as ethanol or the like and surface active agents such as polysorbate 80 (trade name) or the like. Such a composition may further contain additive agents such as an antiseptic, a moistening agent, an emulsifying agent, a dispersing agent, a stabilizing agent (e.g., lactose) and a solubilization assisting agent (e.g., glutamic acid or aspartic acid). These compositions are sterilized by filtration through a bacteria retaining filter, blending of a germicide or irradiation. Alternatively, they may be used by first making it into sterile solid compositions and dissolving them in sterile water or a sterile solvent for injection use prior to their use.

BEST MODE OF CARRYING OUT THE INVENTION

The following illustratively describes the present invention with reference to examples. In this connection, the present invention is not limited by the compounds of the examples. Also, novel materials to be used in the present invention are described as reference examples.

Reference Example 1

2-Bromo-9-tosyl-9H-carbazole

1.32 g (33.0 mmol) of sodium hydride (60% in oil) was added to a solution of 2-bromo-9H-carbazole (7.39 g, 30.0 mmol) in tetrahydrofuran (110 ml), and the mixture was heated under reflux for 30 minutes. After cooling to room temperature, 5.72 g (30.0 mmol) of tosyl chloride was added to this solution, and the mixture was stirred at room temperature for 20 minutes. This solution was poured into 5% aqueous citric acid and extracted with ethyl acetate. The organic layer was washed with water and brine and dried over magnesium sulfate. Then, the solvent was evaporated under reduced pressure to give 12.42 g of crude crystals. The crude crystals were recrystallized from ethyl acetate-n-hexane to give 8.21 g of the title compound as white crystals.

Nuclear magnetic resonance spectrum (DMSO-$d_6$, TMS internal standard)

$\delta$: 2.26 (3H, s), 7.32 (2H, d, J=9Hz), 7.46 (1H, t, J=8Hz), 7.59-7.65 (2H, m), 7.76 (2H, d, J=9Hz), 8.12-8.17 (2H, m), 8.22 (1H, d, J=9Hz), 8.39 (1H, d, J=2Hz).

Reference Example 2

The compound of Reference Example 2 was obtained in the same manner as in Reference Example 1.
9-Benzenesulfonyl-3-bromo-9H-carbazole

Starting compounds: 3-bromo-9H-carbazole and benzenesulfonyl chloride
Nuclear magnetic resonance spectrum (DMSO-$d_6$, TMS internal standard)
δ: 7.45-7.53 (3H, m), 7.61-7.66 (2H, m), 7.74 (1H, dd, J=2Hz, 9Hz), 7.85-7.87 (2H, m), 8.21-8.26 (3H, m), 8.46 (1H, d, J=2Hz).

Reference Example 3

2-Bromo-9-tetrahydropyranyl-9H-carbazole

A mixture of 24.61 g (100 mmol) of 2-bromo-9H-carbazole, 250 ml of methylene chloride, 11.4 ml (125 mmol) of 3,4-dihydro-2H-pyran and 1.16 g (5.0 mmol) of camphorsulfonic acid was stirred at room temperature for 30 minutes. The reaction solution was poured into 1 N aqueous sodium hydroxide and partitioned. The resulting organic layer was washed with water and brine and dried over sodium sulfate. Then, the solvent was evaporated under reduced pressure to give a yellow and oily crude product. The crude product was crystallized from methanol to give 23.80 g of the title compound as white crystals.

Nuclear magnetic resonance spectrum (DMSO-$d_6$, TMS internal standard)
δ: 1.62 (1H, d, J=13Hz), 1.72-1.89 (3H, m), 1.96-1.98 (1H, m), 2.26-2.34 (1H, m), 3.78-3.83 (1H, m), 4.14-4.16 (1H, m), 6.00 (1H, dd, J=2Hz, 11Hz), 7.24 (1H, t, J=7Hz), 7.36 (1H, dd, J=2Hz, 9Hz), 7.45-7.48 (1H, m), 7.80 (1H, d, J=8Hz), 8.00 (1H, d, J=1Hz), 8.10 (1H, d, J=8Hz), 8.16 (1H, d, J=7Hz).

Reference Example 4

The compound of Reference Example 4 was obtained in the same manner as in Reference Example 1.
3-Bromo-9-tosyl-9H-carbazole

Starting compounds: 3-bromo-9H-carbazole and tosyl chloride
Nuclear magnetic resonance spectrum (DMSO-$d_6$, TMS internal standard)
δ: 2.24 (1H, s), 7.28 (2H, d, J=8Hz), 7.46 (1H, t, J=7Hz), 7.62 (1H, t, J=7Hz), 7.72-7.75 (3H, m), 8.07-8.30 (3H, m), 8.45 (1H, d, J=2Hz).

Example 1

a) (9-Tosyl-9H-carbazol-2-yl)(1-trityl-1H-imidazol-4-yl)methanol

A solution of 2-bromo-9-tosyl-9H-carbazole (6.00 g, 15.0 mmol) in tetrahydrofuran (60 ml) was cooled to -70°C under argon atmosphere. n-Butyl lithium (9.2 ml of a 1.64 M solution in hexane, 15.1 mmol) and then a solution of 1-trityl-1H-imidazole-4-carbaldehyde (5.08 g, 15.0 mmol) in tetrahydrofuran (60 ml) were added dropwise to this solution below -70°C. The reaction mixture was allowed to warm to room temperature, then poured into 5% aqueous citric acid and extracted with ethyl acetate. The organic layer was washed with water and brine and dried over magnesium sulfate. Then, the solvent was evaporated under reduced pressure. The resulting residue was purified by silica gel column chromatography (elution with chloroform-ethyl acetate) to give 4.69 g (7.11 mmol) of the title compound as white crystals.

Nuclear magnetic resonance spectrum (DMSO-$d_6$, TMS internal standard)
δ: 2.20 (3H, s), 5.79 (1H, d, J=5Hz), 5.86 (1H, d, J=5Hz), 6.87 (1H, s), 7.12-7.14 (8H, m), 7.34-7.45 (12H, m), 7.54 (1H, t, J=7Hz), 7.64 (2H, d, J=8Hz), 8.02 (1H, d, J=8Hz), 8.07 (1H, d, J=7Hz), 8.23 (1H, d, J=9Hz), 8.27 (1H, s).

b) (9-Tosyl-9H-carbazol-2-yl)(1-trityl-1H-imidazol-4-yl)ketone

A mixture of 4.00 g (6.06 mmol) of (9-tosyl-9H-carbazol-2-yl)(1-trityl-1H-imidazol-4-yl)methanol, 15.1 g (124 mmol) of manganese dioxide (70%) and 80 ml of chloroform was heated under reflux for 16 hours. The reaction mixture was cooled and filtered, and the resulting filtrate was concentrated under reduced pressure and dried. Ethyl acetate (40 ml)

was added to the resulting residue, and the thus precipitated white crystals were collected by filtration and dried to give 2.98 g (4.53 mmol) of the title compound.

Nuclear magnetic resonance spectrum (DMSO-$d_6$, TMS internal standard)
δ: 2.23 (3H, s), 7.21-7.27 (8H, m), 7.42-7.49 (10H, m), 7.64-7.67 (1H, m), 7.74 (1H, s), 7.78-7.83 (3H, m), 8.22-8.30 (4H, m), 9.32 (1H, s).

c) 1-(9-Tosyl-9H-carbazol-2-yl)-1-(1-trityl-1H-imidazol-4-yl)ethanol

Methylmagnesium bromide (1.70 ml of a 3.0 M solution in diethyl ether, 5.10 mmol) was added dropwise to a solution of (9-tosyl-9H-carbazol-2-yl)(1-trityl-1H-imidazol-4-yl)ketone (2.80 g, 4.26 mmol) in tetrahydrofuran (42 ml) under argon atmosphere with ice-cooling. After stirring for 10 minutes, the reaction mixture was poured into 5% aqueous citric acid and extracted with ethyl acetate. The organic layer was washed with water and brine and dried over magnesium sulfate. Then, the solvent was evaporated under reduced pressure. The resulting residue was purified by silica gel column chromatography

(elution with chloroform-hexane → chloroform-ethyl acetate)

to give 1.85 g (2.75 mmol) of the title compound as white crystals.

Nuclear magnetic resonance spectrum (DMSO-$d_6$, TMS internal standard)
δ: 1.81 (3H, s), 2.19 (3H, s), 5.71 (1H, s), 6.87 (1H, s), 7.12-7.14 (8H, m), 7.36-7.44 (11H, m), 7.51-7.59 (2H, m), 7.64 (2H, d, J=8Hz), 7.98 (1H, d, J=8Hz), 8.05 (1H, d, J=8Hz), 8.24 (1H, d, J=8Hz), 8.37 (1H, s).

d) 2-[1-(1H-Imidazol-4-yl)ethyl]-9-tosyl-9H-carbazole

1-(9-Tosyl-9H-carbazol-2-yl)-1-(1-trityl-1H-imidazol-4-yl)ethanol (1.80 g, 2.67 mmol) was dissolved in 18 ml of acetic acid, 180 mg of palladium-carbon (10 %) was added, and the mixture was stirred at 70°C for 16 hours in an atmosphere of hydrogen at 3 atmospheric pressure. After cooling, palladium-carbon was removed by filtration and the filtrate was concentrated under reduced pressure. Ethyl acetate was added to the resulting residue, and the mixture was washed with 1 M aqueous potassium carbonate, water and brine. The organic layer was dried over magnesium sulfate, the solvent was evaporated under reduced pressure, and the resulting residue was purified by silica gel column chromatography (elution with chloroform-methanol-29% aqueous ammonia) to give 1.03 g (2.48 mmol) of the title compound as white foam.

Nuclear magnetic resonance spectrum (DMSO-$d_6$, TMS internal standard)
δ: 1.63 (3H, d, J=7Hz), 2.24 (3H, s), 4.26-4.28 (1H, m), 6.87 (1H, s), 7.24 (2H, d, J=9Hz), 7.33-7.34 (1H, m), 7.40 (1H, t, J=7Hz), 7.53 (1H, t, J=7Hz), 7.62-7.63 (3H, m), 7.98 (1H, d, J=8Hz), 8.05 (1H, d, J=7Hz), 8.10 (1H, s), 8.24 (1H, d, J=9Hz), 11.87 (1H, s).

e) 2-[1-(1H-Imidazol-4-yl)ethyl]-9H-carbazole

A mixture of 1.00 g (2.41 mmol) of 2-[1-(1H-imidazol-4-yl)ethyl]-9-tosyl-9H-carbazole, 30 ml of ethanol and 30 ml of 2 N aqueous sodium hydroxide was heated under reflux for 40 hours. After cooling, the reaction mixture was neutralized by acetic acid (4.1 ml), and the solvent was evaporated under reduced pressure. Ethyl acetate was added to the resulting residue, and the mixture was washed with 1 M aqueous potassium carbonate, water and brine. The organic layer was dried over magnesium sulfate, the solvent was evaporated under reduced pressure, and then the resulting residue was purified by silica gel column chromatography (elution with chloroform-methanol-29% aqueous ammonia) to give 0.41 g (1.57 mmol) of the title compound. By recrystallization from ethyl acetate, white crystals were obtained.

Melting point: 216-218°C
Nuclear magnetic resonance spectrum (DMSO-$d_6$, TMS internal standard)
δ: 1.60 (3H, d, J=7Hz), 4.19-4.21 (1H, m), 6.79 (1H, s), 7.06 (1H, d, J=8Hz), 7.11 (1H, t, J=7Hz), 7.29 (1H, s), 7.32 (1H, t, J=8Hz), 7.43 (1H, d, J=8Hz), 7.51 (1H, s), 7.97 (1H, d, J=8Hz), 8.03 (1H, d, J=7Hz), 11.08 (1H, s), 11.79 (1H, s).

f) 2-[1-(1H-Imidazol-4-yl)ethyl]-9H-carbazole monohydrochloride

2-[1-(1H-Imidazol-4-yl)ethyl]-9H-carbazole (8.6 g, 32.9 mmol) was dissolved in 200 ml of ethanol and, with ice-cooling, 24.7 ml (98.7 mmol) of 4 N hydrogen chloride in ethyl acetate was added. By evaporating the solvent under reduced pressure, 9.9 g of crude crystals were obtained.

The crude crystals were recrystallized from ethanol-ethyl acetate to give 8.69 g of the title compound as white crystals.

Mass spectrometry (m/z): (262 $(M+H)^+$, APCI)

| Elemental analysis (for $C_{17}H_{15}N_3 \cdot HCl$) | | | | |
|---|---|---|---|---|
| | C (%) | H (%) | N (%) | Cl (%) |
| Calcd. | 68.57 | 5.42 | 14.11 | 11.91 |
| Found | 68.39 | 5.49 | 14.02 | 12.07 |

Nuclear magnetic resonance spectrum (DMSO-$d_6$, TMS internal standard)

δ: 1.69 (3H, d, J=7Hz), 4.43 (1H, q, J=7Hz), 7.07 (1H, d, J=8Hz), 7.12-7.15 (1H, m), 7.34-7.37 (2H, m), 7.47 (1H, d, J=8Hz), 7.57 (1H, s), 8.04-8.08 (2H, m), 9.03 (1H, s), 11.28 (1H, s), 14.43 (2H, br).

Example 2

a) 2-(1H-Imidazol-4-ylmethyl)-9-tosyl-9H-carbazole

(9-Tosyl-9H-carbazol-2-yl)(1-trityl-1H-imidazol-4-yl)methanol (530 mg, 0.8 mmol) was dissolved in 16 ml of acetic acid, 60 mg of palladium-carbon (10%) was added, and the mixture was stirred at 70°C for 2 days in an atmosphere of hydrogen at 3 atmospheric pressure. After cooling, palladium-carbon was removed by filtration, the filtrate was concentrated under reduced pressure, and ethyl acetate was added to the residue. The mixture was washed with 1 M aqueous potassium carbonate, water and brine. The organic layer was dried over anhydrous sodium sulfate, the solvent was evaporated under reduced pressure, and the resulting residue was purified by silica gel column chromatography (elution with chloroform-methanol-29% aqueous ammonia) to give 280 mg (0.7 mmol) of the title compound as white foam.

Nuclear magnetic resonance spectrum (DMSO-$d_6$, TMS internal standard)

δ: 2.24 (3H, s), 4.06 (2H, s), 6.84 (1H, br), 7.24 (2H, d, J=9Hz), 7.31-7.33 (1H, m), 7.39-7.41 (1H, m), 7.51-7.55 (1H, m), 7.63-7.67 (3H, m), 7.99 (1H, d, J=8Hz), 8.06 (1H, d, J=8Hz), 8.13 (1H, s), 8.23 (1H, d, J=9Hz), 11.90 (1H, br).

b) 2-(1H-Imidazol-4-ylmethyl)-9H-carbazole

A mixture of 280 mg (0.7 mmol) of 2-(1H-imidazol-4-ylmethyl)-9-tosyl-9H-carbazole, 30 ml of ethanol and 4 ml of 1 N aqueous sodium hydroxide was heated under reflux for 5 hours. After cooling, the reaction mixture was neutralized with acetic acid, and the solvent was evaporated under reduced pressure. Ethyl acetate was added to the residue, and the mixture was washed with 1 M aqueous potassium carbonate, water and brine. The organic layer was dried over anhydrous sodium sulfate, the solvent was evaporated under reduced pressure and then the resulting residue was purified by silica gel column chromatography (elution with chloroform-methanol-29% aqueous ammonia) to give 140 mg (0.57 mmol) of the title compound, which was recrystallized from ethyl acetate to afford white crystals.

Melting point: 230-232°C (decomposition)

Nuclear magnetic resonance spectrum (DMSO-$d_6$, TMS internal standard)

δ: 4.00 (2H, s), 6.78 (1H, s), 7.05 (1H, d, J=8Hz), 7.11 (1H, t, J=7Hz), 7.29-7.34 (2H, m), 7.42-7.44 (1H, m), 7.53 (1H, s), 7.98 (1H, d, J=8Hz), 8.04 (1H, d, J=7Hz), 11.10 (1H, s), 11.82 (1H, br).

c) 2-(1H-Imidazol-4-ylmethyl)-9H-carbazole monohydrochloride

A mixture of 1.02 g (4.12 mmol) of 2-(1H-imidazol-4-ylmethyl)-9H-carbazole and 20 ml of ethanol was heated under reflux to give a homogenous solution. With ice-cooling, 1.6 ml of 4 N hydrogen chloride in ethyl acetate was added to

this solution. The solvent was evaporated under reduced pressure, and the crude crystals thus formed were recrystallized twice from ethanol to give 563 mg of the title compound as white crystals.

| Elemental analysis (for $C_{16}H_{13}N_3 \cdot HCl$) | | | | |
|---|---|---|---|---|
| | C (%) | H (%) | N (%) | Cl (%) |
| Calcd. | 67.72 | 4.97 | 14.81 | 12.49 |
| Found | 67.73 | 5.15 | 14.81 | 12.53 |

Mass spectrometry (m/z): (248 $(M+H)^+$, APCI)
Nuclear magnetic resonance spectrum (DMSO-$d_6$, TMS internal standard)
δ: 4.20 (2H, s), 7.09 (1H, d, J=8Hz), 7.14 (1H, t, J=8Hz), 7.36 (1H, t, J=8Hz), 7.40 (1H, s), 7.46-7.48 (2H, m), 8.04-8.08 (2H, m), 9.04 (1H, d, J=1Hz), 11.34 (1H, s), 15.58 (2H, br).

d) 2-(1H-Imidazol-4-ylmethyl)-9H-carbazole monohydrochloride monohydrate

A mixture of 5.00 g of 2-(1H-imidazol-4-ylmethyl)-9H-carbazole monohydrochloride and 125 ml of water-ethanol (10:1) was stirred at 60°C to give a homogenous solution. This solution was allowed to cool to room temperature with stirring and then stirred overnight at 5°C. The thus precipitated crystals were collected by filtration, washed with 20 ml of cool water and then dried to give 4.49 g of the title compound as white crystals.

| Elemental analysis (for $C_{16}H_{13}N_3 \cdot HCl \cdot H_2O$) | | | | |
|---|---|---|---|---|
| | C (%) | H (%) | N (%) | Cl (%) |
| Calcd. | 63.68 | 5.34 | 13.92 | 11.75 |
| Found | 63.76 | 5.29 | 13.97 | 11.68 |

Mass spectrometry (m/z): 248 ($(M+H)^+$, FAB)
Nuclear magnetic resonance spectrum (DMSO-$d_6$, TMS internal standard)
δ: 4.20 (2H, s), 7.10 (1H, dd, J=1Hz, 8Hz), 7.14 (1H, t, J=8Hz), 7.34-7.38 (1H, m), 7.41 (1H, s), 7.47-7.48 (2H, m), 8.04-8.08 (2H, m), 9.05 (1H, d, J=1Hz), 11.38 (1H, s), 14.66 (2H, br).

Example 3

a) The following compound of Example 3 (a) was obtained in the same manner as in Example 1 (a).

(9-Benzenesulfonyl-9H-carbazol-3-yl)(1-trityl-1H-imidazol-4-yl)methanol

Starting compounds: 9-benzenesulfonyl-3-bromo-9H-carbazole and 1-trityl-1H-imidazole-4-carbaldehyde
Nuclear magnetic resonance spectrum (DMSO-$d_6$, TMS internal standard)
δ: 5.73-5.74 (1H, m), 5.77-5.78 (1H, m), 6.79 (1H, s), 7.06-7.11 (6H, m), 7.28 (1H, d, J=1Hz), 7.36-7.50 (12H, m), 7.54-7.63 (3H, m), 7.84-7.86 (2H, m), 8.05-8.08 (2H, m), 8.17 (1H, d, J=9Hz), 8.26 (1H, d, J=9Hz).

b) The following compound of Example 3 (b) was obtained in the same manner as in Example 1 (d).

9-Benzenesulfonyl-3-(1H-imidazol-4-ylmethyl)-9H-carbazole

Starting compound: (9-benzenesulfonyl-9H-carbazol-3-yl)(1-trityl-1H-imidazol-4-yl)methanol
Nuclear magnetic resonance spectrum (DMSO-$d_6$, TMS internal standard)
δ: 3.99 (2H, s), 6.80 (1H, s), 7.40-7.50 (4H, m), 7.54-7.62 (3H, m), 7.84-7.85 (2H, m), 7.98 (1H, s), 8.07 (1H,

d, J=8Hz), 8.17 (1H, d, J=9Hz), 8.25 (1H, d, J=9Hz), 11.85 (1H, s).

c) The following compound of Example 3 (c) was obtained in the same manner as in Example 1 (e).

3-(1H-Imidazol-4-ylmethyl)-9H-carbazole

Starting compound: 9-benzenesulfonyl-3-(1H-imidazol-4-ylmethyl)-9H-carbazole
Melting point: 218-220°C
Nuclear magnetic resonance spectrum (DMSO-$d_6$, TMS internal standard)
δ: 3.99 (1H, s), 6.72 (1H, s), 7.12 (1H, t, J=7Hz), 7.26 (1H, d, J=8Hz), 7.33-7.39 (2H, m), 7.44 (1H, d, J=8Hz), 7.51 (1H, s), 7.94 (1H, s), 8.04 (1H, d, J=8Hz), 11.11 (1H, s), 11.80 (1H, s).

Example 4

a) The following compound of Example 4 (a) was obtained in the same manner as in Example 1 (a).

(9-Tetrahydropyranyl-9H-carbazol-2-yl)(1-trityl-1H-imidazol-4-yl)methanol

Starting compounds: 2-bromo-9-tetrahydropyranyl-9H-carbazole and 1-trityl-1H-imidazole-4-carbaldehyde
Nuclear magnetic resonance spectrum (DMSO-$d_6$, TMS internal standard)
δ: 1.60-1.69 (3H, m), 1.82-1.85 (1H, m), 2.32-2.37 (1H, m), 3.79 (1H, t, J=9Hz), 4.11 (1H, br), 5.70-5.71 (1H, m), 5.74-5.76 (1H, m), 5.92 (1H, d, J=9Hz), 6.77 (1H, s), 7.08-7.10 (7H, m), 7.16-7.21 (2H, m), 7.28 (1H, d, J=1Hz), 7.35-7.41 (9H, m), 7.73-7.76 (2H, m), 8.02 (1H, d, J=8Hz), 8.08 (1H, d, J=8Hz).

b) The following compound of Example 4 (b) was obtained in the same manner as in Example 1 (b).

(9-Tetrahydropyranyl-9H-carbazol-2-yl)(1-trityl-1H-imidazol-4-yl)ketone

Starting compound: (9-tetrahydropyranyl-9H-carbazol-2-yl)(1-trityl-1H-imidazol-4-yl)methanol
Nuclear magnetic resonance spectrum (DMSO-$d_6$, TMS internal standard)
δ: 1.60-1.99 (5H, m), 2.35-2.38 (1H, m), 3.78-3.82 (1H, m), 4.11-4.13 (1H, m), 6.00-6.03 (1H, m), 7.19 (6H, d, J=7Hz), 7.26 (1H, t, J=7Hz), 7.41-7.52 (10H, m), 7.63 (1H, d, J=1Hz), 7.73 (1H, d, J=1Hz), 7.82 (1H, d, J=9Hz), 8.08-8.09 (1H, m), 8.21-8.25 (2H, m), 8.75 (1H, s).

c) (9H-Carbazol-2-yl)(1H-imidazol-4-yl)ketone

(9-Tetrahydropyranyl-9H-carbazol-2-yl)(1-trityl-1H-imidazol-4-yl)ketone (0.587 g, 1 mmol) was dissolved in 10 ml of dioxane, and 10 ml of 1 N hydrochloric acid was added to the solution at room temperature. The mixture was stirred at 60°C for 2 hours, cooled to room temperature, and diluted by adding 100 ml of ethyl acetate. Then, 100 ml of 3 N hydrochloric acid was added and then stirred. After removing the organic layer, the aqueous layer was neutralized with potassium carbonate, extracted with ethyl acetate and then washed with brine. The organic layer was dried over magnesium sulfate, the solvent was evaporated under reduced pressure and then the resulting residue was recrystallized from methanol to give 0.164 g (0.63 mmol) (63%) of the title compound as yellow crystals.

Melting point: 274°C (decomposition)
Nuclear magnetic resonance spectrum (DMSO-$d_6$, TMS internal standard)
δ: 7.22 (1H, t, J=8Hz), 7.47 (1H, t, J=8Hz), 7.56 (1H, d, J=9Hz), 7.92-7.97 (3H, m), 8.21 (1H, d, J=8Hz), 8.24 (1H, d, J=8Hz), 8.40 (1H, bs), 11.52 (1H, s), 12.90 (1H, bs).

Example 5

a) The following compound of Example 5 (a) was obtained in the same manner as in Example 1 (a).

(9-Ethyl-9H-carbazol-2-yl)(1-trityl-1H-imidazol-4-yl)methanol

Starting compounds: 2-bromo-9-ethyl-9H-carbazole and 1-trityl-1H-imidazole-4-carbaldehyde
Nuclear magnetic resonance spectrum (DMSO-$d_6$, TMS internal standard)
δ: 1.29 (3H, t, J=7Hz), 4.39 (2H, q, J=7Hz), 5.67 (1H, d, J=5Hz), 5.76 (1H, d, J=5Hz), 6.75 (1H, s), 7.08-7.11

(6H, m), 7.13-7.21 (2H, m), 7.28 (1H, s), 7.35-7.43 (10H, m), 7.52 (1H, s), 7.57 (1H, d, J=8Hz), 8.02 (1H, d, J=8Hz), 8.09 (1H, d, J=7Hz).

b) The following compound of Example 5 (b) was obtained in the same manner as in Example 1 (d).

9-Ethyl-2-(1H-imidazol-4-ylmethyl)-9H-carbazole

Starting compound: (9-ethyl-9H-carbazol-2-yl)(1-trityl-1H-imidazol-4-yl)methanol
Melting point: 173-174°C
Nuclear magnetic resonance spectrum (DMSO-$d_6$, TMS internal standard)
δ: 1.30 (3H, t, J=7Hz), 4.04 (2H, s), 4.39 (2H, q, J=7Hz), 6.77 (1H, s), 7.08 (1H, d, J=8Hz), 7.14-7.18 (1H, m), 7.38-7.43 (1H, m), 7.46 (1H, s), 7.54-7.57 (2H, m), 8.02 (1H, d, J=8Hz), 8.08 (1H, d, J=8Hz), 11.85 (1H, bs).

Example 6

a) 2-[(1-Dimethylcarbamoyl-1H-imidazol-4-yl)methyl]-9H-carbazole

A mixture of 2-(1H-imidazol-4-ylmethyl)-9H-carbazole (950 mg, 3.9 mmol), anhydrous dimethylformamide (20 ml), triethylamine (580 mg, 5.7 mmol) and N,N-dimethylcarbamoyl chloride (620 mg, 5.8 mmol) was heated overnight at 50°C. After cooling of the reaction mixture, water was added, and the mixture was extracted with ethyl acetate. The organic layer was washed with brine and dried over anhydrous sodium sulfate, and then the solvent was evaporated under reduced pressure. The resulting residue was purified by silica gel column chromatography (chloroform:methanol:28% aqueous ammonia = 50:1:0.1) to give 2-[(1-dimethylcarbamoyl-1H-imidazol-4-yl)methyl]-9H-carbazole (950 mg), which was recrystallized from ethyl acetate to afford white crystals.

Nuclear magnetic resonance spectrum (DMSO-$d_6$, TMS internal standard)
δ: 3.00 (6H, s), 3.98 (2H, s), 7.07 (1H, d, J=8Hz), 7.12 (1H, t, J=7Hz), 7.22 (1H, s), 7.32-7.34 (2H, m), 7.44 (1H, d, J=8Hz), 7.99 (2H, d, J=6Hz), 8.04 (1H, d, J=7Hz), 11.11 (1H, s).

b) 2-[(1-Ethyl-1H-imidazol-5-yl)methyl]-9H-carbazole

A mixture of 2-[(1-dimethylcarbamoyl-1H-imidazol-4-yl)methyl]-9H-carbazole (510 mg, 1.6 mmol), anhydrous dimethylformamide (10 ml) and ethyl iodide (5.0 g, 32 mmol) was heated at 100°C for 4 days. After cooling of the reaction mixture, ethyl acetate saturated with ammonia gas (30 ml) was added. The mixture was stirred at room temperature for 1 hour, water was added and the mixture was extracted with ethyl acetate. The organic layer was washed with brine and dried over anhydrous sodium sulfate. The solvent was evaporated under reduced pressure and then the residue was purified by silica gel column chromatography (chloroform:methanol:28% aqueous ammonia = 30:1:0.1) to give 2-[(1-ethyl-1H-imidazol-5-yl)methyl]-9H-carbazole (110 mg), which was recrystallized from ethyl acetate to afford white crystals.

Melting point: 205-206°C
Nuclear magnetic resonance spectrum (DMSO-$d_6$, TMS internal standard)
δ: 1.13 (3H, t, J=7Hz), 3.81 (2H, q, J=7Hz), 4.11 (2H, s), 6.70 (1H, s), 7.02 (1H, d, J=8Hz), 7.11-7.14 (1H, m), 7.25 (1H, s), 7.33-7.36 (1H, m), 7.44 (1H, d, J=8Hz), 7.59 (1H, s), 8.02 (1H, d, J=8Hz), 8.05 (1H, d, J=8Hz).

Example 7

a) 1-(9H-Fluoren-2-yl)-1-(1-methyl-2-phenylthio-1H-imidazol-5-yl)ethanol

A mixture of 2,2,6,6-tetramethylpiperidine (2.05 g, 14.5 mmol), tetrahydrofuran (50 ml) and ethylene glycol dimethyl ether (25 ml) was cooled to -78°C under argon atmosphere, n-butyl lithium (1.6 M n-hexane solution, 9 ml) was added dropwise to the solution, and the mixture was stirred at the same temperature for 20 minutes. A solution of 1-methyl-2-phenylthio-1H-imidazole (2.5 g, 13.2 mmol) in tetrahydrofuran (25 ml) was added dropwise to this solution, and the mixture was stirred at the same temperature for 1 hour. A solution of 2-acetyl-9H-fluorene (3.0 g, 14.5 mmol) in tetrahydrofuran (50 ml) was added dropwise, and the mixture was allowed to warm to room temperature and stirred overnight. A saturated aqueous ammonium chloride was added to the reaction mixture, and the mixture was extracted with ethyl acetate. The organic layer was washed with brine and dried over anhydrous sodium sulfate, and then the solvent was evaporated under reduced pressure. The resulting residue was purified by silica gel column chromatography (n-hexane:ethyl acetate = 2:1) to give 1-(9H-fluoren-2-yl)-1-(1-methyl-2-phenylthio-1H-imidazol-5-yl)ethanol (1.7 g), which was recrystallized from ethyl acetate to afford white crystals.

Nuclear magnetic resonance spectrum (DMSO-$d_6$, TMS internal standard)

δ: 1.84 (3H, s), 3.20 (3H, s), 3.85 (1H, d, J=22Hz), 3.91 (1H, d, J=22Hz), 6.09 (1H, s), 7.02 (2H, d, J=8Hz), 7.17-7.20 (1H, m), 7.28-7.31 (5H, m), 7.35-7.38 (1H, m), 7.50 (1H, s), 7.56 (1H, d, J=7Hz), 7.82 (1H, d, J=8Hz), 7.85 (1H, d, J=7Hz).

b) 1-(9H-Fluoren-2-yl)-1-(1-methyl-1H-imidazol-5-yl)ethanol

A mixture of 1-(9H-fluoren-2-yl)-1-(1-methyl-2-phenylthio-1H-imidazol-5-yl)ethanol (590 mg, 1.5 mmol), Raney nickel (Raney NDHT-90: manufactured by Kawaken Fine Chemical) (about 4.5 g) and ethanol (60 ml) was heated under reflux for 2 hours. The reaction mixture was cooled to room temperature and filtered. The resulting filtrate was concentrated under reduced pressure, and the residue was purified by silica gel column chromatography (chloroform:methanol = 50:1) to give 1-(9H-fluoren-2-yl)-1-(1-methyl-1H-imidazol-5-yl)ethanol (310 mg), which was crystallized and washed with diethyl ether to afford white crystals.

Melting point: 264-265°C

Nuclear magnetic resonance spectrum (DMSO-$d_6$, TMS internal standard)

δ: 1.81 (3H, s), 3.23 (3H, s), 3.88 (2H, s), 5.90 (1H, s), 7.03 (1H, s), 7.27-7.30 (2H, m), 7.36 (1H, t, J=7Hz), 7.51 (2H, s), 7.56 (1H, d, J=7Hz), 7.81 (1H, d, J=8Hz), 7.84 (1H, d, J=7Hz).

c) 5-[1-(9H-Fluoren-2-yl)vinyl]-1-methyl-1H-imidazole (7c1) and 5-[1-(9H-fluoren-2-yl)ethyl]-1-methyl-1H-imidazole (7c2)

Under argon atmosphere, triethylsilyl hydride (550 mg, 4.7 mmol) was added dropwise to a mixture of 1-(9H-fluoren-2-yl)-1-(1-methyl-1H-imidazol-5-yl)ethanol (230 mg, 0.8 mmol) and trifluoroacetic acid (3 ml) with ice-cooling, and the mixture was stirred for 1 hour at the same temperature. The reaction mixture was concentrated under reduced pressure, saturated aqueous sodium bicarbonate was added, and the mixture was extracted with ethyl acetate. The organic layer was washed with brine and dried over anhydrous sodium sulfate. Then, the solvent was evaporated under reduced pressure. The resulting residue was purified by silica gel column chromatography (chloroform:methanol:28% aqueous ammonia = 200:1:0.1) to give 5-[1-(9H-fluoren-2-yl)vinyl]-1-methyl-1H-imidazole (170 mg) and 5-[1-(9H-fluoren-2-yl)ethyl]-1-methyl-1H-imidazole (30 mg). They were respectively recrystallized from ethyl acetate to give respective white crystals.

5-[1-(9H-Fluoren-2-yl)vinyl]-1-methyl-1H-imidazole

Melting point: 150°C

5-[1-(9H-Fluoren-2-yl)ethyl]-1-methyl-1H-imidazole

Melting point: 161-162°C

Nuclear magnetic resonance spectrum ($CDCl_3$, TMS internal standard)

5-[1-(9H-Fluoren-2-yl)vinyl]-1-methyl-1H-imidazole

δ: 3.29 (3H, s), 3.89 (2H, s), 5.43 (1H, d, J=1Hz), 5.70-5.72 (1H, m), 7.13 (1H, s), 7.30-7.40 (3H, m), 7.47 (2H, d, J=3Hz), 7.55 (1H, d, J=7Hz), 7.74 (1H, d, J=8Hz), 7.78 (1H, d, J=7Hz).

5-[1-(9H-Fluoren-2-yl)ethyl]-1-methyl-1H-imidazole

δ: 1.56 (3H, d, J=7Hz), 3.29 (3H, s), 3.87 (2H, s), 4.19-4.24 (1H, m), 6.92 (1H, s), 7.19 (1H, d, J=7Hz), 7.28 (1H, t, J=7Hz), 7.34-7.37 (2H, m), 7.51-7.56 (2H, m), 7.80-7.85 (2H, m).

Example 8

a) The following compound of Example 8 (a) was obtained in the same manner as in Example 7 (a).

(9H-Fluoren-2-yl)(1-methyl-2-phenylthio-1H-imidazol-5-yl)methanol

Starting compounds: 1-methyl-2-phenylthio-1H-imidazole and 9H-fluorene-2-carbaldehyde

Nuclear magnetic resonance spectrum (DMSO-$d_6$, TMS internal standard)

δ: 3.55 (3H, s), 3.88 (2H, s), 5.93 (1H, s), 6.91 (1H, s), 7.15-7.56 (10H, m), 7.73-7.81 (2H, m).

b) The following compound of Example 8 (b) was obtained in the same manner as in Example 7 (b).

(9H-Fluoren-2-yl)(1-methyl-1H-imidazol-5-yl)methanol

Starting compound: (9H-fluoren-2-yl)(1-methyl-2-phenylthio-1H-imidazol-5-yl)methanol
Melting point: 232-233°C
Nuclear magnetic resonance spectrum (DMSO-$d_6$, TMS internal standard)
δ: 3.56 (3H, s), 3.92 (2H, s), 5.86 (1H, d, J=5Hz), 5.94 (1H, d, J=5Hz), 6.47 (1H, s), 7.29-7.32 (1H, m), 7.37-7.41 (2H, m), 7.53 (1H, s), 7.57-7.60 (2H, m), 7.87 (2H, t, J=7Hz).

c) The following compound of Example 8 (c) was obtained in the same manner as in Example 7 (c).

5-(9H-Fluoren-2-ylmethyl)-1-methyl-1H-imidazole

Starting compound: (9H-fluoren-2-yl)(1-methyl-1H-imidazol-5-yl)methanol
Melting point: 155-156°C
Nuclear magnetic resonance spectrum ($CDCl_3$, TMS internal standard)
δ: 3.44 (3H, s), 3.85 (2H, s), 4.01 (2H, s), 6.91 (1H, s), 7.17 (1H, d, J=8Hz), 7.26-7.30 (2H, m), 7.36 (1H, t, J=7Hz), 7.49-7.53 (2H, m), 7.70 (1H, d, J=8Hz), 7.75 (1H, d, J=7Hz).

Example 9

a) The following compound of Example 9 (a) was obtained in the same manner as in Example 7 (a).

(1-Ethyl-2-phenylthio-1H-imidazol-5-yl)(9H-fluoren-2-yl)methanol

Starting compounds: 1-ethyl-2-phenylthio-1H-imidazole and 9H-fluorene-2-carbaldehyde
Nuclear magnetic resonance spectrum ($CDCl_3$, TMS internal standard)
δ: 1.09 (3H, t, J=7Hz), 3.88 (2H, s), 4.03-4.11 (2H, m), 5.93 (1H, s), 6.91 (1H, s), 7.15-7.26 (5H, m), 7.30-7.41 (3H, m), 7.53-7.58 (2H, m), 7.77-7.79 (2H, m).

b) The following compound of Example 9 (b) was obtained in the same manner as in Example 7 (b).

(1-Ethyl-1H-imidazol-5-yl)(9H-fluoren-2-yl)methanol

Starting compound: (1-ethyl-2-phenylthio-1H-imidazol-5-yl)(9H-fluoren-2-yl)methanol
Melting point: 188-189°C
Nuclear magnetic resonance spectrum ($CDCl_3$, TMS internal standard)
δ: 1.28 (3H, t, J=7Hz), 3.87 (2H, s), 3.88-3.99 (2H, m), 5.94 (1H, s), 6.70 (1H, s), 7.29-7.41 (4H, m), 7.52-7.57 (2H, m), 7.74-7.78 (2H, m).

c) The following compound of Example 9 (c) was obtained in the same manner as in Example 7 (c).

1-Ethyl-5-(9H-fluoren-2-ylmethyl)-1H-imidazole

Starting compound: (1-ethyl-1H-imidazol-5-yl)(9H-fluoren-2-yl)methanol
Melting point: 158-159°C
Nuclear magnetic resonance spectrum ($CDCl_3$, TMS internal standard)
δ: 1.25 (3H, t, J=7Hz), 3.74-3.79 (2H, m), 3.85 (2H, s), 4.02 (2H, s), 6.89 (1H, s), 7.18 (1H, d, J=7Hz), 7.26-7.38 (3H, m), 7.48-7.53 (2H, m), 7.70 (1H, d, J=7Hz), 7.75 (1H, d, J=8Hz).

Example 10

a) The following compound of Example 10 (a) was obtained in the same manner as in Example 7 (a).

1-(1-Ethyl-2-phenylthio-1H-imidazol-5-yl)-1-(9H-fluoren-2-yl)ethanol

Starting compounds: 1-ethyl-2-phenylthio-1H-imidazole and 2-acetyl-9H-fluorene

Nuclear magnetic resonance spectrum ($CDCl_3$, TMS internal standard)

δ: 0.80 (3H, t, J=7Hz), 1.98 (3H, s), 3.67-3.74 (1H, m), 3.83 (1H, d, J=22Hz), 3.89 (1H, d, J=22Hz), 3.94-4.01 (1H, m), 7.15-7.20 (2H, m), 7.23-7.26 (3H, m), 7.28-7.38 (4H, m), 7.51-7.54 (2H, m), 7.71 (1H, d, J=8Hz), 7.76 (1H, d, J=7Hz).

b) The following compound of Example 10 (b) was obtained in the same manner as in Example 7 (b).

1-(1-Ethyl-1H-imidazol-5-yl)-1-(9H-fluoren-2-yl)ethanol

Starting compound: 1-(1-ethyl-2-phenylthio-1H-imidazol-5-yl)-1-(9H-fluoren-2-yl)ethanol
Nuclear magnetic resonance spectrum (DMSO-$d_6$, TMS internal standard)

δ: 0.90 (3H, t, J=7Hz), 1.81 (3H, s), 3.68-3.73 (2H, m), 3.88 (2H, s), 5.96 (1H, s), 7.01 (1H, s), 7.27-7.30 (2H, m), 7.36 (1H, t, J=7Hz), 7.51-7.59 (3H, m), 7.80 (1H, d, J=8Hz), 7.84 (1H, d, J=7Hz).

c) 1-Ethyl-5-[1-(9H-fluoren-2-yl)vinyl]-1H-imidazole

A mixture of 1-(1-ethyl-1H-imidazol-5-yl)-1-(9H-fluoren-2-yl)ethanol (80 mg, 0.26 mmol) and trifluoroacetic acid (4 ml) was stirred for 1 hour with ice-cooling and then warmed to room temperature and stirred for 4 hours. The reaction mixture was concentrated under reduced pressure, saturated aqueous sodium bicarbonate was added, and the mixture was extracted with ethyl acetate. The resulting organic layer was washed with brine and dried over anhydrous sodium sulfate. Then, the solvent was evaporated under reduced pressure. The resulting residue was purified by silica gel column chromatography (chloroform:methanol = 100:1) to give 1-ethyl-5-[1-(9H-fluoren-2-yl)vinyl]-1H-imidazole (70 mg), which was crystallized and washed with n-hexane to afford white crystals.

Melting point: 128-129°C
Nuclear magnetic resonance spectrum ($CDCl_3$, TMS internal standard)

δ: 1.20 (3H, t, J=7Hz), 3.61 (2H, q, J=7Hz), 3.88 (2H, s), 5.44 (1H, d, J=1Hz), 5.70 (1H, d, J=1Hz), 7.13 (1H, s), 7.30-7.40 (3H, m), 7.47 (1H, s), 7.54-7.55 (2H, m), 7.73 (1H, d, J=8Hz), 7.78 (1H, d, J=8Hz).

d) 1-Ethyl-5-[1-(9H-fluoren-2-yl)ethyl]-1H-imidazole

1-Ethyl-5-[1-(9H-fluoren-2-yl)vinyl]-1H-imidazole (690 mg, 2.4 mmol) was dissolved in 10 ml of methanol, 70 mg of palladium-carbon (10%) was added, and the mixture was stirred at room temperature under hydrogen atmosphere for 3 days. After removal of palladium-carbon by filtration, the resulting filtrate was concentrated under reduced pressure and the resulting residue was purified by silica gel column chromatography (elution with chloroform-methanol) to give 120 mg (0.4 mmol) of the title compound as white crystals. Melting point: 134-135°C

Nuclear magnetic resonance spectrum (DMSO-$d_6$, TMS internal standard)

δ: 1.01 (3H, t, J=7Hz), 1.56 (3H, d, J=6Hz), 3.63-3.71 (2H, m), 3.87 (2H, s), 4.19-4.23 (1H, m), 6.93 (1H, s), 7.20 (1H, d, J=8Hz), 7.27-7.30 (1H, m), 7.34-7.37 (2H, m), 7.54-7.58 (2H, m), 7.80-7.84 (2H, m).

Example 11

a) The following compound of Example 11 (a) was obtained in the same manner as in Example 7 (a).

(9-Benzenesulfonyl-9H-carbazol-2-yl)(1-methyl-2-phenylthio-1H-imidazol-5-yl)methanol

Starting compounds: 1-methyl-2-phenylthio-1H-imidazole and 9-benzenesulfonyl-9H-carbazole-2-carbaldehyde
Nuclear magnetic resonance spectrum (DMSO-$d_6$, TMS internal standard)

δ: 3.56 (3H, s), 6.09 (1H, d, J=6Hz), 6.35 (1H, d, J=6Hz), 6.63 (1H, s), 7.12 (2H, d, J=7Hz), 7.22 (1H, t, J=7Hz), 7.30-7.34 (2H, m), 7.43-7.47 (4H, m), 7.58 (1H, t, J=7Hz), 7.62 (1H, t, J=7Hz), 7.76 (2H, d, J=8Hz), 8.11-8.13 (2H, m), 8.27 (1H, d, J=8Hz), 8.35 (1H, s).

b) The following compound of Example 11 (b) was obtained in the same manner as in Example 7 (b).

(9-Benzenesulfonyl-9H-carbazol-2-yl)(1-methyl-1H-imidazol-5-yl)methanol

Starting compound: (9-benzenesulfonyl-9H-carbazol-2-yl)(1-methyl-2-phenylthio-1H-imidazol-5-yl)methanol

Nuclear magnetic resonance spectrum (DMSO-$d_6$, TMS internal standard)

δ: 3.32 (3H, s), 6.22 (1H, bs), 6.42 (1H, bs), 7.43-7.45 (5H, m), 7.56-7.62 (3H, m), 7.74-7.76 (2H, m), 8.10-8.11 (2H, m), 8.26 (1H, d, J=8Hz), 8.35 (1H, bs).

c) 9-Benzenesulfonyl-2-[ethoxy(1-methyl-1H-imidazol-5-yl)methyl]-9H-carbazole

Triethylsilane (3.44 ml, 21.54 mmol) and trifluoroborane etherate (1.32 ml, 10.77 mmol) were added to 160 ml of chloroform solution containing 1.500 g (3.59 mmol) of (9-benzenesulfonyl-9H-carbazol-2-yl)(1-methyl-1H-imidazol-5-yl)methanol, and the mixture was stirred at room temperature for 1 hour. Then, 3.44 ml (21.54 mmol) of triethylsilane and 1.32 ml (10.77 mmol) of trifluoroborane etherate were added again, followed by 1 hour of stirring at the same temperature. The reaction mixture was heated to 50°C and stirred for 18 hours, 1.00 ml (6.26 mmol) of triethylsilane and 0.50 ml (4.08 mmol) of trifluoroborane etherate were added, and then the mixture was stirred for additional 1 hour. The reaction mixture was washed with saturated aqueous sodium bicarbonate and brine, and the organic layer was dried over magnesium sulfate. The solvent was evaporated under reduced pressure, and the resulting residue was purified by silica gel column chromatography. The crude product was obtained from a fraction eluted with chloroform-methanol (100:1) and then recrystallized from ethyl acetate to give 0.768 g (1.72 mmol) (48%) of the title compound as white crystals.

Nuclear magnetic resonance spectrum (DMSO-$d_6$, TMS internal standard)

δ: 1.24 (3H, t, J=7Hz), 3.47 (1H, dq, J=7Hz, 7Hz), 3.57 (3H, s), 3.58 (1H, dq, J=7Hz, 7Hz), 5.82 (1H, s), 6.37 (1H, s), 7.44-7.49 (4H, m), 7.59-7.63 (3H, m), 7.65 (1H, s), 7.72 (2H, d, J=7Hz), 8.14 (2H, d, J=8Hz), 8.28 (1H, s), 8.30 (1H, d, J=8Hz).

d) 9-Benzenesulfonyl-2-[(1-methyl-1H-imidazol-5-yl)methyl]-9H-carbazole

9-Benzenesulfonyl-2-[ethoxy(1-methyl-1H-imidazol-5-yl)methyl]-9H-carbazole (0.730 g, 1.64 mmol) was dissolved in 60 ml of acetic acid, 1.200 g of palladium-carbon (10%) was added, and the mixture was stirred at 70°C for 19 hours under 4 atmospheric pressure of hydrogen. The reaction mixture was cooled to room temperature and filtered. The resulting filtrate was concentrated under reduced pressure. Chloroform was added to the resulting residue, the mixture was washed with saturated aqueous sodium bicarbonate and brine, and then the organic layer was dried over magnesium sulfate. The solvent was evaporated under reduced pressure and the resulting residue was purified by silica gel column chromatography to give 0.470 g (1.17 mmol) (71%) of the title compound as white crystals.

Nuclear magnetic resonance spectrum (DMSO-$d_6$, TMS internal standard)

δ: 3.41 (3H, s), 4.18 (2H, s), 6.72 (1H, s), 7.28 (1H, d, J=8Hz), 7.43 (1H, t, J=7Hz), 7.47 (2H, t, J=8Hz), 7.56 (1H, t, J=8Hz), 7.60 (1H, s), 7.62 (1H, t, J=7Hz), 7.71-7.73 (2H, m), 8.04 (1H, s), 8.05 (1H, d, J=8Hz), 8.09 (1H, d, J=7Hz), 8.26 (1H, d, J=9Hz).

e) The following compound of Example 11 (e) was obtained in the same manner as in Example 1 (e).

2-[(1-Methyl-1H-imidazol-5-yl)methyl]-9H-carbazole

Starting compound: 9-benzenesulfonyl-2-[(1-methyl-1H-imidazol-5-yl)methyl]-9H-carbazole
Melting point: 242.5-244°C
Nuclear magnetic resonance spectrum (DMSO-$d_6$, TMS internal standard)

δ: 3.44 (3H, s), 4.10 2H, s), 6.71 (1H, s), 7.01 (1H, d, J=8Hz), 7.13 (1H, t, J=8Hz), 7.23 (1H, s), 7.34 (1H, t, J=8Hz), 7.44 (1H, d, J=8Hz), 7.52 (1H, s), 8.02 (1H, d, J=8Hz), 8.05 (1H, d, J=8Hz), 11.13 (1H, s).

Example 12

a) Diethyl (9-benzenesulfonyl-9H-carbazol-2-yl)methylphosphonate

A mixture of 9-benzenesulfonyl-2-bromomethyl-9H-carbazole (833 mg, 2.08 mmol) and triethyl phosphite (0.36 ml, 2.08 mmol) was stirred at 80°C for 5 days. The reaction mixture was concentrated under reduced pressure and purified by silica gel column chromatography (eluent; chloroform:methanol = 10:1) to give the title compound (526 mg, 1.19 mmol, 43%) as colorless crystals.

Nuclear magnetic resonance spectrum ($CDCl_3$, TMS internal standard)

δ: 1.25 (6H, t, J=7Hz), 3.36 (2H, d, J=22Hz), 4.04 (4H, dq, J=7Hz, 7Hz), 7.26-7.50 (6H, m), 7.79-7.90 (4H, m), 8.29-8.36 (2H, m).

b) (E)-9-Benzenesulfonyl-2-[2-(1-trityl-1H-imidazol-4-yl)ethenyl]-9H-carbazole

Sodium hydride (60 wt%, 49 mg, 1.22 mmol) was added at 0°C to a mixture of diethyl (9-benzenesulfonyl-9H-carbazol-2-yl)methylphosphonate (515 mg, 1.16 mmol), 1-trityl-1H-imidazole-4-carbaldehyde (397 mg, 1.16 mmol), a catalytic amount of 15-crown-5-ether and THF (12 ml), and the resulting mixture was heated under reflux for 5 hours. Brine (10 ml) was added to the reaction mixture, and the product was extracted with ethyl acetate. The extract was dried over anhydrous sodium sulfate and concentrated under reduced pressure to give the title compound (703 mg, 1.10 mmol, 94%) as colorless crystals.

Nuclear magnetic resonance spectrum ($CDCl_3$, TMS internal standard)
δ: 6.96 (1H, d, J=1Hz), 7.13-7.54 (24H, m), 7.75-7.85 (3H, m), 8.30 (1H, d, J=8Hz), 8.43 (1H, s).

c) (E)-9-Benzenesulfonyl-2-[2-(1H-imidazol-4-yl)ethenyl]-9H-carbazole

A mixture of (E)-9-benzenesulfonyl-2-[2-(1-trityl-1H-imidazol-4-yl)ethenyl]-9H-carbazole (693 mg, 1.08 mmol) and 90% aqueous acetic acid (20 ml) was stirred at 60°C for 2 hours. The reaction mixture was concentrated under reduced pressure, ethyl acetate (20 ml) and saturated aqueous sodium bicarbonate (20 ml) were added to the resulting residue, and then the product was extracted with ethyl acetate. The extract was washed with brine, dried over anhydrous magnesium sulfate and then concentrated under reduced pressure. The resulting residue was purified by silica gel column chromatography (eluent; chloroform:methanol:17% ammonia = 100:3:0.3) to give the title compound (387 mg, 0.97 mmol, 90%) as colorless crystals.

Nuclear magnetic resonance spectrum ($CDCl_3$, TMS internal standard)
δ: 7.16-7.49 (9H, m), 7.55 (1H, s), 7.65 (1H, s), 7.76-7.88 (3H, m), 8.28 (1H, dd, J=2Hz, 7Hz), 8.42 (1H, s).

d) 9-Benzenesulfonyl-2-[2-(1H-imidazol-4-yl)ethyl]-9H-carbazole

A mixture of (E)-9-benzenesulfonyl-2-[2-(1H-imidazol-4-yl)ethenyl]-9H-carbazole (387 mg, 0.97 mmol), a catalytic amount of palladium-carbon (10%) and dioxane (8 ml) was stirred at room temperature for 3 days under hydrogen atmosphere. The reaction mixture was filtered and the resulting filtrate was concentrated to give the title compound (322 mg, 0.80 mmol, 83%) as colorless crystals.

Nuclear magnetic resonance spectrum ($CDCl_3$, TMS internal standard)
δ: 2.89-3.23 (4H, m), 6.77-6.83 (1H, m), 7.24-8.29 (13H, m)

e) 2-[2-(1H-imidazol-4-yl)ethyl]-9H-carbazole

A mixture of 9-benzenesulfonyl-2-[2-(1H-imidazol-4-yl)ethyl]-9H-carbazole (322 mg, 0.80 mmol), ethanol (15 ml) and 2 N aqueous sodium hydroxide (7.5 ml) was heated under reflux for 12 hours. The reaction mixture was concentrated under reduced pressure, and purified by silica gel column chromatography (eluent; chloroform:methanol:17% ammonia = 100:10:1) and then recrystallized from ethanol to give the title compound (110 mg, 0.27 mmol, 34%) as colorless crystals.

Melting point: 239-241°C
Nuclear magnetic resonance spectrum (DMSO-$d_6$, TMS internal standard)
δ: 2.82-2.93 (2H, m), 3.02-3.07 (2H, m), 6.74 (1H, s), 7.03 (1H, d, J=8Hz), 7.10-7.14 (1H, m), 7.29-7.35 (2H, m), 7.44 (1H, d, J=8Hz), 7.52 (1H, s), 7.97-8.05 (2H, m), 11.11 (1H, br), 11.71 (1H, br).

Example 13

a) The following compound of Example 13 was obtained in the same manner as in Example 6 (b).

2-[(1-Propyl-1H-imidazol-5-yl)methyl]-9H-carbazole

Starting compounds: 2-[(1-dimethylcarbamoyl-1H-imidazol-4-yl)methyl]-9H-carbazole and propyl iodide

Melting point: 189-191°C

Nuclear magnetic resonance spectrum (DMSO-$d_6$, TMS internal standard)

δ: 0.73 (3H, t, J=7Hz), 1.36-1.53 (2H, m), 3.74 (2H, t, J=7Hz), 4.10 (2H, s), 6.70 (1H, s), 7.01-7.02 (1H, m), 7.13 (1H, t, J=7Hz), 7.25 (1H, s), 7.34 (1H, dd, J=7Hz, 7Hz), 7.44 (1H, d, J=8Hz), 7.56 (1H, s), 8.01 (1H, d, J=8Hz), 8.05 (1H, d, J=8Hz), 11.13 (1H, s).

Example 14

a) The following compound was obtained in the same manner as in Example 1 (a).

(1-Isopropyl-1H-imidazol-5-yl)(9-tosyl-9H-carbazol-2-yl)methanol

Starting compounds: 2-bromo-9-tosyl-9H-carbazole and 1-isopropyl-1H-imidazole-5-carbaldehyde

Nuclear magnetic resonance spectrum (DMSO-$d_6$, TMS internal standard)

δ: 1.12 (3H, d, J=7Hz), 1.33 (3H, d, J=6Hz), 2.26 (3H, s), 4.44-4.50 (1H, m), 6.04 (1H, d, J=5Hz), 6.24 (1H, d, J=5Hz), 6.47 (1H, s), 7.25 (2H, d, J=8Hz), 7.40-7.45 (2H, m), 7.54-7.59 (1H, m), 7.62 (2H, d, J=8Hz), 7.81 (1H, s), 8.07-8.12 (2H, m), 8.26 (1H, d, J=8Hz), 8.33 (1H, s).

b) 2-[(1-Isopropyl-1H-imidazol-5-yl)methyl]-9-tosyl-9H-carbazole

(1-Isopropyl-1H-imidazol-5-yl)(9-tosyl-9H-carbazol-2-yl)methanol (1.56 g, 3.4 mmol) was dissolved in 20 ml of trifluoroacetic acid, 2.4 g (20.4 mmol) of triethylsilyl hydride was added, and the mixture was stirred for 1 hour with ice-cooling. The solvent was evaporated under reduced pressure, and ethyl acetate was added to the residue, followed by washing with saturated aqueous sodium bicarbonate, water and brine. The organic layer was dried over anhydrous sodium sulfate, the solvent was evaporated under reduced pressure, and then the resulting residue was recrystallized from ethyl acetate to give 1.36 g (3.1 mmol) of the title compound as white crystals.

Nuclear magnetic resonance spectrum (DMSO-$d_6$, TMS internal standard)

δ: 1.18 (6H, d, J=7Hz), 2.25 (3H, s), 4.19 (2H, s), 4.22-4.25 (1H, m), 6.72 (1H, s), 7.24 (2H, d, J=8Hz), 7.29 (1H, d, J=8Hz), 7.42 (1H, t, J=7Hz), 7.55 (1H, t, J=7Hz), 7.60 (2H, d, J=8Hz), 7.79 (1H, s), 8.04 (2H, d, J=7Hz), 8.08 (1H, d, J=7Hz), 8.24 (1H, d, J=8Hz).

c) The following compound was obtained in the same manner as in Example 1 (e).

2-[(1-Isopropyl-1H-imidazol-5-yl)methyl]-9H-carbazole

Starting compound: 2-[(1-isopropyl-1H-imidazol-5-yl)methyl]-9-tosyl-9H-carbazole

Melting point: 181-182°C

Nuclear magnetic resonance spectrum (DMSO-$d_6$, TMS internal standard)

δ: 1.23 (6H, d, J=7Hz), 4.12 (2H, s), 4.18-4.23 (1H, m), 6.70 (1H, s), 7.01 (1H, d, J=8Hz), 7.11-7.15 (1H, m), 7.23 (1H, s), 7.32-7.36 (1H, m), 7.44 (1H, d, J=8Hz), 7.73 (1H, s), 8.01 (1H, d, J=8Hz), 8.05 (1H, d, J=8Hz), 11.13 (1H, s).

The following compound of Example 15 (a) was obtained in the same manner as in Example 1 (a).

Example 15

a) (1-Benzyl-1H-imidazol-5-yl)(9-tosyl-9H-carbazol-2-yl)methanol

Starting compounds: 2-bromo-9-tosyl-9H-carbazole and 1-benzyl-1H-imidazole-5-carbaldehyde

Nuclear magnetic resonance spectrum (DMSO-$d_6$, TMS internal standard)

δ: 1.99 (3H, s), 5.18 (1H, d, J=15Hz), 5.26 (1H, d, J=15Hz), 5.86 (1H, d, J=6Hz), 6.24 (1H, d, J=6Hz), 6.44 (1H, s), 7.09 (2H, d, J=7Hz), 7.21-7.29 (6H, m), 7.41-7.44 (1H, m), 7.55-7.58 (1H, m), 7.61 (2H, d, J=8Hz), 7.68 (1H, s), 8.01 (1H, d, J=8Hz), 8.09 (1H, d, J=7Hz), 8.24-8.27 (2H, m).

The following compound of Example 15 (b) was obtained in the same manner as in Example 14 (b).

b) 2-[(1-Benzyl-1H-imidazol-5-yl)methyl]-9-tosyl-9H-carbazole

Starting compound: (1-benzyl-1H-imidazol-5-yl)(9-tosyl-9H-carbazol-2-yl)methanol
Nuclear magnetic resonance spectrum (DMSO-$d_6$, TMS internal standard)

δ: 2.22 (3H, s), 4.04 (2H, s), 5.07 (2H, s), 6.74 (1H, s), 7.00-7.01 (2H, m), 7.15-7.17 (1H, m), 7.20-7.26 (5H, m), 7.40-7.43 (1H, m), 7.53-7.57 (1H, m), 7.61 (2H, d, J=9Hz), 7.76 (1H, s), 7.96-8.00 (2H, m), 8.07 (1H, d, J=7Hz), 8.24 (1H, d, J=9Hz).

The following compound of Example 15 (c) was obtained in the same manner as in Example 1 (e).

c) 2-[(1-Benzyl-1H-imidazol-5-yl)methyl]-9H-carbazole

Starting compound: 2-[(1-benzyl-1H-imidazol-5-yl)methyl]-9-tosyl-9H-carbazole
Melting point: 237-238°C
Nuclear magnetic resonance spectrum (DMSO-$d_6$, TMS internal standard)

δ: 3.95 (2H, s), 5.06 (2H, s), 6.72 (1H, s), 6.93 (1H, d, J=8Hz), 7.06 (2H, d, J=7Hz), 7.12-7.15 (1H, m), 7.18 (1H, s), 7.25-7.28 (1H, m), 7.31-7.36 (3H, m), 7.44 (1H, d, J=8Hz), 7.70 (1H, s), 8.00 (1H, d, J=8Hz), 8.06 (1H, d, J=8Hz), 11.16 (1H, s).

The following compound of Example 16 (a) was obtained in the same manner as in Example 1 (c).

Example 16

a) 1-(9-Tosyl-9H-carbazol-2-yl)-1-(1-trityl-1H-imidazol-4-yl)propanol

Starting compounds: (9-tosyl-9H-carbazol-2-yl)(1-trityl-1H-imidazol-4-yl)ketone and ethylmagnesium bromide
Nuclear magnetic resonance spectrum (DMSO-$d_6$, TMS internal standard)

δ: 0.71-0.75 (3H, m), 1.16-1.19 (2H, m), 2.19 (3H, s), 5.49 (1H, s), 6.87 (1H, s), 7.10-7.12 (8H, m), 7.36-7.43 (11H, m), 7.51-7.55 (1H, m), 7.61-7.67 (3H, m), 7.97 (1H, d, J=8Hz), 8.05 (1H, d, J=8Hz), 8.25 (1H, d, J=8Hz), 8.49 (1H, s).

The following compound of Example 16 (b) was obtained in the same manner as in Example 1 (d).

b) 2-[1-(1H-Imidazol-4-yl)propyl]-9-tosyl-9H-carbazole

Starting compound: 1-(9-tosyl-9H-carbazol-2-yl)-1-(1-trityl-1H-imidazol-4-yl)propanol
Nuclear magnetic resonance spectrum (DMSO-$d_6$, TMS internal standard)

δ: 0.85 (3H, t, J=7Hz), 1.97-1.99 (1H, m), 2.14-2.20 (1H, m), 2.24 (3H, s), 4.02-4.04 (1H, m), 6.90 (1H, br), 7.22 (2H, d, J=8Hz), 7.33 (1H, d, J=7Hz), 7.39-7.42 (1H, m), 7.51-7.54 (1H, m), 7.62-7.64 (3H, m), 7.98 (1H, br), 8.05 (1H, d, J=7Hz), 8.17 (1H, br), 8.23-8.25 (1H, m), 11.81 (1H, br).

The following compound of Example 16 (c) was obtained in the same manner as in Example 1 (e).

c) 2-[1-(1H-Imidazol-4-yl)propyl]-9H-carbazole

Starting compound: 2-[1-(1H-imidazol-4-yl)propyl]-9-tosyl-9H-carbazole
Melting point: 229-230°C
Nuclear magnetic resonance spectrum (DMSO-$d_6$, TMS internal standard)

δ: 0.84 (3H, t, J=7Hz), 1.91-1.99 (1H, m), 2.08-2.15 (1H, m), 3.89 (1H, br), 6.81 (1H, s), 7.07-7.12 (2H, m), 7.31-7.34 (2H, m), 7.43 (1H, d, J=8Hz), 7.50 (1H, s), 7.96 (1H, d, J=8Hz), 8.03 (1H, d, J=8Hz), 11.07 (1H, s), 11.77 (1H, br).

The following compound of Example 17 (a) was obtained in the same manner as in Example 1 (c).

Example 17

a) 1-(9-Tosyl-9H-carbazol-2-yl)-1-(1-trityl-1H-imidazol-4-yl)butanol

Starting compounds: (9-tosyl-9H-carbazol-2-yl)(1-trityl-1H-imidazol-4-yl)ketone and propylmagnesium bromide
Nuclear magnetic resonance spectrum (DMSO-$d_6$, TMS internal standard)
δ: 0.80-0.83 (3H, m), 1.08-1.11 (1H, m), 1.23-1.28 (1H, m), 2.04-2.08 (1H, m), 2.13-2.15 (1H, m), 2.20 (3H, s), 5.49 (1H, s), 6.85 (1H, s), 7.10-7.13 (8H, m), 7.37-7.42 (11H, m), 7.51-7.56 (1H, m), 7.60-7.65 (3H, m), 7.96 (1H, d, J=9Hz), 8.04 (1H, d, J=7Hz), 8.24 (1H, d, J=8Hz), 8.47 (1H, s).

The following compound of Example 17 (b) was obtained in the same manner as in Example 1 (d).

b) 2-[1-(1H-Imidazol-4-yl)butyl]-9-tosyl-9H-carbazole

Starting compound: 1-(9-tosyl-9H-carbazol-2-yl)-1-(1-trityl-1H-imidazol-4-yl)butanol
Nuclear magnetic resonance spectrum (DMSO-$d_6$, TMS internal standard)
δ: 0.91 (3H, t, J=7Hz), 1.14-1.25 (2H, m), 1.94 (1H, br), 2.11 (1H, br), 2.23 (3H, s), 4.01-4.05 (1H, m), 6.91 (1H, s), 7.22 (2H, d, J=8Hz), 7.33-7.35 (1H, m), 7.39-7.42 (1H, m), 7.51-7.54 (1H, m), 7.62-7.64 (3H, m), 7.94-7.96 (1H, m), 8.04-8.05 (1H, m), 8.19-8.26 (2H, m), 11.81 (1H, br).

The following compound of Example 17 (c) was obtained in the same manner as in Example 1 (e).

c) 2-[1-(1H-Imidazol-4-yl)butyl]-9H-carbazole

Starting compound: 2-[1-(1H-imidazol-4-yl)butyl]-9-tosyl-9H-carbazole
Melting point: 220°C
Nuclear magnetic resonance spectrum (DMSO-$d_6$, TMS internal standard)
δ: 0.88 (3H, t, J=7Hz), 1.17-1.28 (2H, m), 1.87-1.95 (1H, m), 2.04-2.11 (1H, m), 3.99-4.02 (1H, m), 6.81 (1H, s), 7.07-7.12 (2H, m), 7.31-7.34 (2H, m), 7.43 (1H, d, J=9Hz), 7.50 (1H, s), 7.96 (1H, d, J=8Hz), 8.03 (1H, d, J=8Hz), 11.07 (1H, s), 11.78 (1H, br).

The following compound of Example 18 (a) was obtained in the same manner as in Example 1 (c).

Example 18

a) 2-Methyl-1-(9-tosyl-9H-carbazol-2-yl)-1-(1-trityl-1H-imidazol-4-yl)propanol

Starting compounds: (9-tosyl-9H-carbazol-2-yl)(1-trityl-1H-imidazol-4-yl)ketone and isopropylmagnesium chloride
Nuclear magnetic resonance spectrum (DMSO-$d_6$, TMS internal standard)
δ: 0.63 (3H, d, J=7Hz), 0.82 (3H, d, J=7Hz), 2.19 (3H, s), 2.65-2.68 (1H, m), 5.30 (1H, s), 6.92 (1H, s), 7.07-7.10 (7H, m), 7.20-7.22 (1H, m), 7.38-7.48 (11H, m), 7.51-7.55 (1H, m), 7.69 (2H, d, J=8Hz), 7.74 (1H, d, J=8Hz), 7.95 (1H, d, J=8Hz), 8.04 (1H, d, J=8Hz), 8.26 (1H, d, J=8Hz), 8.69 (1H, s).

The following compound of Example 18 (b) was obtained in the same manner as in Example 1 (d).

b) 2-[1-(1H-Imidazol-4-yl)-2-methylpropyl]-9-tosyl-9H-carbazole

Starting compound: 2-methyl-1-(9-tosyl-9H-carbazol-2-yl)-1-(1-trityl-1H-imidazol-4-yl)propanol
Nuclear magnetic resonance spectrum (DMSO-$d_6$, TMS internal standard)
δ: 0.76 (3H, d, J=7Hz), 0.92 (3H, d, J=6Hz), 3.67 (1H, br), 6.96 (1H, s), 7.20 (2H, d, J=8Hz), 7.38-7.41 (2H, m), 7.51-7.55 (1H, m), 7.63-7.68 (3H, m), 7.95 (1H, d, J=8Hz), 8.03 (1H, d, J=8Hz), 8.25 (1H, d, J=8Hz), 8.32 (1H, s), 11.80 (1H, br).

The following compound of Example 18 (c) was obtained in the same manner as in Example 1 (e).

c) 2-[1-(1H-Imidazol-4-yl)-2-methylpropyl]-9H-carbazole

Starting compound: 2-[1-(1H-imidazol-4-yl)-2-methylpropyl]-9-tosyl-9H-carbazole

Mass spectrometry (m/z): 289 ($M^+$, EI)
Nuclear magnetic resonance spectrum (DMSO-$d_6$, TMS internal standard)
δ: 0.75 (3H, d, J=7Hz), 0.89 (3H, d, J=6Hz), 2.41-2.47 (1H, m), 3.60 (1H, d, J=10Hz), 6.88 (1H, s), 7.08-7.16 (2H, m), 7.30-7.34 (1H, m), 7.41-7.43 (2H, m), 7.51 (1H, s), 7.94 (1H, d, J=8Hz), 8.02 (1H, d, J=8Hz), 11.08 (1H, s), 11.79 (1H, br).

Example 19

a) 1-(1H-Imidazol-4-yl)-2-methyl-1-(9-tosyl-9H-carbazol-2-yl)propanol

2-Methyl-1-(9-tosyl-9H-carbazol-2-yl)-1-(1-trityl-1H-imidazol-4-yl)propanol (1.89 g, 2.7 mmol) was dissolved in 90% aqueous acetic acid and the mixture was heated at 60°C for 3 hours. After cooling, the solvent was evaporated under reduced pressure, ethyl acetate was added to the resulting residue, and the mixture was extracted with 1 N hydrochloric acid. The water layer was neutralized with saturated aqueous sodium bicarbonate, extracted with ethyl acetate and then washed with water and brine. The organic layer was dried over anhydrous sodium sulfate, the solvent was evaporated under reduced pressure, and then the resulting residue was purified by silica gel column chromatography (elution with chloroform-methanol-29% aqueous ammonia) to give 1.08 g (2.4 mmol) of the title compound as white foam.

Nuclear magnetic resonance spectrum ($CDCl_3$, TMS internal standard)
δ: 0.79 (3H, d, J=7Hz), 1.02 (3H, d, J=7Hz), 2.21 (3H, s), 2.57-2.86 (1H, m), 6.95-7.08 (3H, m), 7.31-7.47 (2H, m), 7.55-7.64 (4H, m), 7.75-7.84 (2H, m), 8.28-8.35 (1H, m), 8.49 (1H, s).

b) 2-[1-(1H-Imidazol-4-yl)-2-methyl-1-propenyl]-9-tosyl-9H-carbazole

1-(1H-Imidazol-4-yl)-2-methyl-1-(9-tosyl-9H-carbazol-2-yl)propanol (1.08 g, 2.4 mmol) was dissolved in 50 ml of methylene chloride, 3 g (24 mmol) of trifluoroacetic acid was added with ice-cooling, and then the mixture was stirred at room temperature for 4 days. The solvent was evaporated under reduced pressure, ethyl acetate was added to the residue, and the mixture was washed with saturated aqueous sodium bicarbonate, water and brine. The organic layer was dried over anhydrous sodium sulfate, the solvent was evaporated under reduced pressure, and then the resulting residue was purified by silica gel column chromatography (elution with chloroform-methanol-29% aqueous ammonia) to give 950 mg (2.2 mmol) of the title compound as white foam.

Nuclear magnetic resonance spectrum (DMSO-$d_6$, TMS internal standard)
δ: 1.72 (3H, br), 2.15 (3H, br), 2.26 (3H, s), 6.23 (1H, br), 7.16-7.18 (1H, m), 7.27 (2H, d, J=8Hz), 7.43 (1H, t, J=7Hz), 7.54-7.62 (4H, m), 7.95 (1H, s), 8.05 (1H, d, J=8Hz), 8.10 (1H, d, J=7Hz), 8.26 (1H, d, J=8Hz), 12.01 (1H, br).

c) 2-[1-(1H-Imidazol-4-yl)-2-methyl-1-propenyl]-9H-carbazole monohydrochloride

A mixture of 2-[1-(1H-imidazol-4-yl)-2-methyl-1-propenyl]-9-tosyl-9H-carbazole (950 mg, 2.15 mmol), ethanol (100 ml) and 2 N aqueous potassium hydroxide (11 ml, 21.5 mmol) was heated under reflux for 14 hours. After cooling, the reaction mixture was neutralized with acetic acid (2.6 g, 43 mmol), and the solvent was evaporated under reduced pressure. Ethyl acetate was added to the residue and the mixture was washed with 1 M aqueous potassium carbonate, water and brine. The organic layer was dried over anhydrous sodium sulfate, the solvent was evaporated under reduced pressure, and then the residue was purified by silica gel column chromatography (elution with chloroform-methanol-29% aqueous ammonia) to give 550 mg of a white foam. This was dissolved in ethyl acetate, 1 ml of 4 N hydrogen chloride in ethyl acetate was added with ice-cooling, and the thus precipitated crystals were collected by filtration to give 480 mg (1.5 mmol) of the title compound as white crystals.

Melting point: 165-167°C
Nuclear magnetic resonance spectrum (DMSO-$d_6$, TMS internal standard)
δ: 1.86 (3H, s), 1.95 (3H, s), 6.92-6.94 (1H, m), 7.14-7.17 (1H, m), 7.27 (1H, s), 7.36-7.40 (1H, m), 7.49 (1H, d, J=8Hz), 7.59 (1H, s), 8.08-8.11 (2H, m), 9.01 (1H, s), 11.38 (1H, s), 14.40 (2H, br).

The following compound of Example 20 (a) was obtained in the same manner as in Example 1 (c).

Example 20

a) Phenyl(9-tosyl-9H-carbazol-2-yl)(1-trityl-1H-imidazol-4-yl)methanol

Starting compounds: (9-tosyl-9H-carbazol-2-yl)(1-trityl-1H-imidazol-4-yl)ketone and phenylmagnesium bromide
Nuclear magnetic resonance spectrum (DMSO-$d_6$, TMS internal standard)
δ: 2.22 (3H, s), 6.35 (1H, s), 6.81 (1H, s), 7.15-7.18 (8H, m), 7.22-7.24 (1H, m), 7.28-7.32 (2H, m), 7.37-7.47 (14H, m), 7.52-7.57 (3H, m), 7.95 (1H, d, J=8Hz), 8.04 (1H, d, J=8Hz), 8.24-8.27 (2H, m).

The following compound of Example 20 (b) was obtained in the same manner as in Example 1 (d).

b) 2-[(1H-Imidazol-4-yl)(phenyl)methyl]-9-tosyl-9H-carbazole

Starting compound: phenyl(9-tosyl-9H-carbazol-2-yl)(1-trityl-1H-imidazol-4-yl)methanol
Nuclear magnetic resonance spectrum (DMSO-$d_6$, TMS internal standard)
δ: 2.24 (3H, s), 5.63 (1H, s), 6.72 (1H, br), 7.20-7.27 (3H, m), 7.32-7.42 (6H, m), 7.53-7.55 (3H, m), 7.72 (1H, s), 7.98 (1H, d, J=8Hz), 8.05 (1H, d, J=7Hz), 8.13 (1H, br), 8.26 (1H, d, J=9Hz), 11.97 (1H, br).

The following compound of Example 20 (c) was obtained in the same manner as in Example 19 (c).

c) 2-[(1H-Imidazol-4-yl)(phenyl)methyl]-9H-carbazole monohydrochloride

Starting compound: 2-[1-(1H-imidazol-4-yl)(phenyl)methyl]-9-tosyl-9H-carbazole
Melting point: 182-187°C
Nuclear magnetic resonance spectrum (DMSO-$d_6$, TMS internal standard)
δ: 5.87 (1H, s), 7.03-7.05 (1H, m), 7.13-7.18 (2H, m), 7.28-7.31 (4H, m), 7.35-7.39 (3H, m), 7.46-7.48 (1H, m), 8.07-8.09 (2H, m), 9.13 (1H, s), 11.34 (1H, s), 14.62 (2H, br).

The following compound of Example 21 (a) was obtained in the same manner as in Example 1 (a).

Example 21

a) (9-Tosyl-9H-carbazol-3-yl)(1-trityl-1H-imidazol-4-yl)methanol

Starting compounds: 3-bromo-9-tosyl-9H-carbazole and 1-trityl-1H-imidazole-4-carbaldehyde
Nuclear magnetic resonance spectrum (DMSO-$d_6$, TMS internal standard)
δ: 2.23 (3H, s), 5.73 (1H, d, J=5Hz), 5.76 (1H, d, J=5Hz), 6.79 (1H, s), 7.09-7.11 (6H, m), 7.25-7.28 (3H, m), 7.35-7.45 (10H, m), 7.53-7.58 (2H, m), 7.73 (2H, d, J=9Hz), 8.04-8.07 (2H, m), 8.16 (1H, d, J=9Hz), 8.25 (1H, d, J=8Hz).

The following compound of Example 21 (b) was obtained in the same manner as in Example 1 (b).

b) (9-Tosyl-9H-carbazol-3-yl)(1-trityl-1H-imidazol-4-yl)ketone

Starting compound: (9-tosyl-9H-carbazol-3-yl)(1-trityl-1H-imidazol-4-yl)methanol
Nuclear magnetic resonance spectrum (DMSO-$d_6$, TMS internal standard)
δ: 2.24 (3H, s), 7.18-7.20 (6H, m), 7.30 (2H, d, J=9Hz), 7.40-7.49 (10H, m), 7.61-7.64 (1H, m), 7.69 (1H, d, J=1Hz), 7.75 (1H, d, J=1Hz), 7.81 (2H, d, J=9Hz), 8.19 (1H, d, J=8Hz), 8.29 (1H, d, J=9Hz), 8.37 (1H, d, J=9Hz), 8.48-8.50 (1H, m), 9.00 (1H, d, J=1Hz).

The following compound of Example 21 (c) was obtained in the same manner as in Example 1 (c).

c) 1-(9-Tosyl-9H-carbazol-3-yl)-1-(1-trityl-1H-imidazol-4-yl)ethanol

Starting compounds: (9-tosyl-9H-carbazol-3-yl)(1-trityl-1H-imidazol-4-yl)ketone and methylmagnesium bromide
Nuclear magnetic resonance spectrum (DMSO-$d_6$, TMS internal standard)
δ: 1.80 (3H, s), 2.23 (3H, s), 5.70 (1H, s), 6.81 (1H, s), 7.09-7.11 (6H, m), 7.26 (1H, d, J=8Hz), 7.35-7.45 (11H, m), 7.54-7.58 (1H, m), 7.63-7.66 (1H, m), 7.74 (2H, d, J=8Hz), 8.05 (1H, d, J=8Hz), 8.11-8.13 (2H, m), 8.25

(1H, d, J=8Hz).

The following compound of Example 21 (d) was obtained in the same manner as in Example 1 (d).

d) 3-[1-(1H-Imidazol-4-yl)ethyl]-9-tosyl-9H-carbazole

Starting compound: 1-(9-tosyl-9H-carbazol-3-yl)-1-(1-trityl-1H-imidazol-4-yl)ethanol
Nuclear magnetic resonance spectrum (DMSO-$d_6$, TMS internal standard)
δ: 1.60 (3H, d, J=7Hz), 4.21 (1H, q, J=7Hz), 6.80 (1H, s), 7.26 (2H, d, J=8Hz), 7.39-7.46 (2H, m), 7.53-7.57 (2H, m), 7.73 (2H, d, J=8Hz), 8.00 (1H, d, J=1Hz), 8.08 (1H, d, J=7Hz), 8.14 (1H, d, J=9Hz), 8.25 (1H, d, J=9Hz), 11.82 (1H, s).

The following compound of Example 21 (e) was obtained in the same manner as in Example 1 (e).

e) 3-[1-(1H-Imidazol-4-yl)ethyl]-9H-carbazole

Starting compound: 3-[1-(1H-imidazol-4-yl)ethyl]-9-tosyl-9H-carbazole
Melting point: 197-198°C
Nuclear magnetic resonance spectrum (DMSO-$d_6$, TMS internal standard)
δ: 1.62 (3H, d, J=7Hz), 4.20 (1H, q, J=7Hz), 6.74 (1H, s), 7.12 (1H, t, J=7Hz), 7.26-7.28 (1H, m), 7.32-7.38 (2H, m), 7.44 (1H, d, J=8Hz), 7.50 (1H, s), 7.96 (1H, s), 8.04 (1H, d, J=8Hz), 11.10 (1H, s), 11.76 (1H, s).

The following compound of Example 22 (a) was obtained in the same manner as in Example 1 (a).

Example 22

a) (1-Methyl-1H-imidazol-5-yl)(9-tosyl-9H-carbazol-2-yl)methanol

Starting compounds: 2-bromo-9-tosyl-9H-carbazole and 1-methyl-1H-imidazole-5-carbaldehyde
Nuclear magnetic resonance spectrum (DMSO-$d_6$, TMS internal standard)
δ: 2.25 (3H, s), 3.54 (3H, s), 6.05 (1H, d, J=5Hz), 6.19 (1H, d, J=5Hz), 6.44 (1H, s), 7.26 (2H, d, J=8Hz), 7.42-7.45 (2H, m), 7.56-7.64 (4H, m), 8.08-8.16 (2H, m), 8.27 (1H, d, J=8Hz), 8.33 (1H, s).

The following compound of Example 22 (b) was obtained in the same manner as in Example 1 (b).

b) (1-Methyl-1H-imidazol-5-yl)(9-tosyl-9H-carbazol-2-yl)ketone

Starting compound: (1-methyl-1H-imidazol-5-yl)(9-tosyl-9H-carbazol-2-yl)methanol
Nuclear magnetic resonance spectrum (DMSO-$d_6$, TMS internal standard)
δ: 2.26 (3H, s), 4.00 (3H, s), 7.32 (2H, d, J=8Hz), 7.48-7.52 (1H, m), 7.59 (1H, s), 7.65-7.68 (1H, m), 7.75 (2H, d, J=8Hz), 7.91 (1H, d, J=8Hz), 8.11 (1H, s), 8.26-8.29 (2H, m), 8.32 (1H, d, J=8Hz), 8.71 (1H, s).

The following compound of Example 22 (c) was obtained in the same manner as in Example 1 (c).

c) 1-(1-Methyl-1H-imidazol-5-yl)-1-(9-tosyl-9H-carbazol-2-yl)ethanol

Starting compounds: (1-methyl-1H-imidazol-5-yl)(9-tosyl-9H-carbazol-2-yl)ketone and methylmagnesium bromide
Nuclear magnetic resonance spectrum (DMSO-$d_6$, TMS internal standard)
δ: 1.88 (3H, s), 2.25 (3H, s), 3.15 (3H, s), 6.22 (1H, s), 7.21 (1H, s), 7.26 (2H, d, J=8Hz), 7.31-7.33 (1H, m), 7.43 (1H, t, J=7Hz), 7.51-7.58 (3H, m), 7.75 (1H, s), 8.03 (1H, d, J=9Hz), 8.08 (1H, d, J=8Hz), 8.25-8.28 (2H, m).

The following compound of Example 22 (d) was obtained in the same manner as in Example 1 (d).

d) 2-[1-(1-Methyl-1H-imidazol-5-yl)ethyl]-9-tosyl-9H-carbazole

Starting compound: 1-(1-methyl-1H-imidazol-5-yl)-1-(9-tosyl-9H-carbazol-2-yl)ethanol
Nuclear magnetic resonance spectrum (DMSO-$d_6$, TMS internal standard)
δ: 1.61 (3H, d, J=7Hz), 2.25 (3H, s), 3.19 (3H, s), 4.37-4.42 (1H, m), 7.00 (1H, s), 7.24-7.27 (3H, m), 7.42

(1H, t, J=7Hz), 7.50-7.57 (3H, m), 7.61 (1H, s), 7.94 (1H, s), 8.03 (1H, d, J=8Hz), 8.07 (1H, d, J=7Hz), 8.26 (1H, d, J=9Hz).

The following compound of Example 22 (e) was obtained in the same manner as in Example 1 (e).

e) 2-[1-(1-Methyl-1H-imidazol-5-yl)ethyl]-9H-carbazole

Starting compound: 2-[1-(1-methyl-1H-imidazol-5-yl)ethyl]-9-tosyl-9H-carbazole
Melting point: 204-205°C
Nuclear magnetic resonance spectrum (DMSO-$d_6$, TMS internal standard)
δ: 1.60 (3H, d, J=7Hz), 3.25 (3H, s), 4.27-4.31 (1H, m), 6.94 (1H, s), 6.98-7.00 (1H, m), 7.11-7.14 (1H, m), 7.16 (1H, s), 7.32-7.36 (1H, m), 7.43 (1H, d, J=9Hz), 7.50 (1H, s), 8.00-8.05 (2H, m), 11.11 (1H, s).

The following compound of Example 23 (a) was obtained in the same manner as in Example 1 (a).

Example 23

a) (9-Methyl-9H-carbazol-2-yl)(1-trityl-1H-imidazol-4-yl)methanol

Starting compounds: 2-bromo-9-methyl-9H-carbazole and 1-trityl-1H-imidazole-4-carbaldehyde
Nuclear magnetic resonance spectrum (DMSO-$d_6$, TMS internal standard)
δ: 3.83 (3H, s), 5.68-5.70 (1H, br), 5.76-5.77 (1H, m), 6.78 (1H, s), 7.09-7.20 (8H, m), 7.28 (1H, s), 7.35-7.45 (9H, m), 7.52 (1H, s), 7.56 (1H, d, J=8Hz), 8.03 (1H, d, J=8Hz), 8.09 (1H, d, J=8Hz), 8.32 (1H, s).

The following compound of Example 23 (b) was obtained in the same manner as in Example 1 (b).

b) (9-Methyl-9H-carbazol-2-yl)(1-trityl-1H-imidazol-4-yl)ketone

Starting compound: (9-methyl-9H-carbazol-2-yl)(1-trityl-1H-imidazol-4-yl)methanol
Nuclear magnetic resonance spectrum (DMSO-$d_6$, TMS internal standard)
δ: 3.91 (3H, s), 7.19-7.30 (7H, m), 7.41-7.48 (9H, m), 7.53-7.56 (1H, m), 7.65-7.66 (2H, m), 7.75 (1H, s), 8.08 (1H, d, J=9Hz), 8.22-8.26 (2H, m), 8.52 (1H, s).

The following compound of Example 23 (c) was obtained in the same manner as in Example 1 (c).

c) 1-(9-Methyl-9H-carbazol-2-yl)-1-(1-trityl-1H-imidazol-4-yl)ethanol

Starting compounds: (9-methyl-9H-carbazol-2-yl)(1-trityl-1H-imidazol-4-yl)ketone and methylmagnesium bromide
Nuclear magnetic resonance spectrum (DMSO-$d_6$, TMS internal standard)
δ: 1.82 (3H, s), 3.82 (3H, s), 5.53 (1H, br), 6.75 (1H, s), 7.08-7.10 (6H, m), 7.14-7.18 (1H, m), 7.28-7.31 (2H, m), 7.34-7.44 (10H, m), 7.56 (1H, d, J=8Hz), 7.60 (1H, s), 7.99 (1H, d, J=8Hz), 8.07 (1H, d, J=8Hz).

The following compound of Example 23 (d) was obtained in the same manner as in Example 1 (d).

d) 2-[1-(1-1H-Imidazol-4-yl)ethyl]-9-methyl-9H-carbazole

Starting compound: 1-(9-methyl-9H-carbazol-2-yl)-1-(1-trityl-1H-imidazol-4-yl)ethanol
Melting point: 215°C (decomposition)
Nuclear magnetic resonance spectrum (DMSO-$d_6$, TMS internal standard)
δ: 1.64 (3H, d, J=7Hz), 3.83 (3H, s), 4.25 (1H, q, J=7Hz), 6.78 (1H, s), 7.10 (1H, d, J=8Hz), 7.16 (1H, t, J=7Hz), 7.40-7.43 (1H, m), 7.45 (1H, s), 7.52-7.55 (2H, m), 8.01 (1H, d, J=8Hz), 8.07 (1H, d, J=8Hz), 11.82 (1H, br).

The following compound of Example 24 (a) was obtained in the same manner as in Example 1 (b).

Example 24

a) (1-Benzyl-1H-imidazol-5-yl)(9-tosyl-9H-carbazol-2-yl)ketone

Starting compound: (1-benzyl-1H-imidazol-5-yl)(9-tosyl-9H-carbazol-2-yl)methanol
Nuclear magnetic resonance spectrum (DMSO-$d_6$, TMS internal standard)
δ: 2.26 (3H, s), 5.69 (2H, s), 7.25-7.31 (5H, m), 7.35-7.38 (2H, m), 7.48-7.51 (1H, m), 7.64-7.71 (4H, m), 7.85-7.87 (1H, m), 8.24-8.30 (3H, m), 8.34 (1H, s), 8.61 (1H, s).

The following compound of Example 24 (b) was obtained in the same manner as in Example 1 (c).

b) 1-(1-Benzyl-1H-imidazol-5-yl)-1-(9-tosyl-9H-carbazol-2-yl)ethanol

Starting compounds: (1-benzyl-1H-imidazol-5-yl)(9-tosyl-9H-carbazol-2-yl)ketone and methylmagnesium bromide
Nuclear magnetic resonance spectrum (DMSO-$d_6$, TMS internal standard)
δ: 1.90 (3H, s), 2.13 (3H, s), 4.72 (1H, d, J=15Hz), 4.91 (1H, d, J=15Hz), 6.30 (1H, s), 6.74 (2H, d, J=7Hz), 6.96-7.04 (3H, m), 7.11 (2H, d, J=9Hz), 7.17 (1H, s), 7.22-7.24 (1H, m), 7.42 (1H, t, J=8Hz), 7.46 (1H, s), 7.54-7.57 (3H, m), 7.91 (1H, d, J=8Hz), 8.05 (1H, d, J=8Hz), 8.26 (1H, d, J=8Hz), 8.32 (1H, s).

c) 2-[1-(1-Benzyl-1H-imidazol-5-yl)vinyl]-9-tosyl-9H-carbazole

1-(1-Benzyl-1H-imidazol-5-yl)-1-(9-tosyl-9H-carbazol-2-yl)ethanol (810 mg, 1.6 mmol) was dissolved in 10 ml of toluene, 1.8 g (16 mmol) of trifluoroacetic acid was added to the solution, and the mixture was heated under reflux for 14 hours. After cooling, water was added and the mixture was extracted with ethyl acetate. The organic layer was washed with water and brine and dried over anhydrous sodium sulfate. Then, the solvent was evaporated under reduced pressure. The resulting residue was purified by silica gel column chromatography (elution with chloroform-methanol) to give 570 mg (1.1 mmol) of the title compound as white foam.

Nuclear magnetic resonance spectrum (DMSO-$d_6$, TMS internal standard)
δ: 2.25 (3H, s), 4.87 (2H, s), 5.42 (1H, s), 5.71 (1H, s), 6.91 (2H, d, J=7Hz), 7.04 (1H, s), 7.19-7.26 (3H, m), 7.29 (2H, d, J=7Hz), 7.32-7.34 (1H, m), 7.43-7.46 (1H, m), 7.57-7.60 (3H, m), 7.96-7.99 (2H, m), 8.09 (1H, d, J=8Hz), 8.14 (1H, d, J=8Hz), 8.25 (1H, d, J=9Hz).

The following compound of Example 24 (d) was obtained in the same manner as in Example 19 (c).

d) 2-[1-(1-Benzyl-1H-imidazol-5-yl)vinyl]-9H-carbazole monohydrochloride

Starting compound: 2-[1-(1-benzyl-1H-imidazol-5-yl)vinyl]-9-tosyl-9H-carbazole
Melting point: 137-140°C
Nuclear magnetic resonance spectrum (DMSO-$d_6$, TMS internal standard)
δ: 5.08 (2H, s), 5.52 (1H, s), 6.01 (1H, s), 7.03-7.05 (2H, m), 7.09-7.10 (1H, m), 7.17-7.20 (1H, m), 7.26-7.31 (4H, m), 7.39-7.43 (1H, m), 7.51 (1H, d, J=8Hz), 7.92 (1H, d, J=2Hz), 8.11-8.14 (2H, m), 9.45 (1H, s), 11.37 (1H, s).

Example 25

4-(9H-Fluoren-2-ylmethyl)-1H-imidazole

A chloroform (100 ml) solution containing 10 g (71 mmol) of 1-trimethylsilyl-1H-imidazole was added to another chloroform (200 ml) solution containing 13.5 g (71 mmol) of titanium tetrachloride with ice-cooling, and the mixture was stirred at room temperature for 30 minutes. A chloroform (100 ml) solution containing 3 g (14 mmol) of 2-chloromethyl-9H-fluorene was added, and the mixture was stirred at room temperature for 14 hours. The reaction mixture was poured into ice-water and extracted with methylene chloride. The organic layer was dried over anhydrous sodium sulfate, the solvent was evaporated under reduced pressure, and the resulting residue was purified by silica gel column chromatography (elution with chloroform-methanol-29% aqueous ammonia) to give 180 mg (0.7 mmol) of the title compound, which was recrystallized from ethyl acetate to afford white crystals.

Melting point: 205-206°C
Nuclear magnetic resonance spectrum (DMSO-$d_6$, TMS internal standard)

δ: 3.86 (2H, s), 3.91 (2H, s), 6.77 (1H, s), 7.25-7.29 (2H, m), 7.34-7.37 (1H, m), 7.43 (1H, s), 7.53-7.56 (2H, m), 7.78 (1H, d, J=8Hz), 7.83 (1H, d, J=7Hz), 11.83 (1H, br).

Chemical structures of the compounds obtained in the Examples are shown in Table 2.

In the table, the binding position "a" means binding position of A on the imidazole ring, and the binding position "b" means binding position of A on the ring

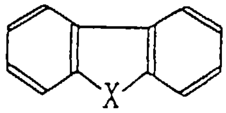

.

Also, abbreviations in the table represent the following groups.

Me --- methyl
Et --- ethyl
n-Pr --- normal propyl
iso-Pr --- isopropyl
n-Bu --- normal butyl

Table 2

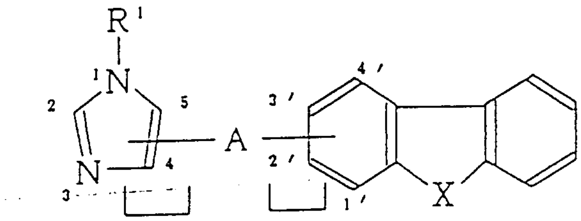

(I)

Bonding position a Bonding position b

| Example | $R^1$ | Bonding position a | A | Bonding position b | X | Salt, etc. |
|---|---|---|---|---|---|---|
| 1 e | H | 4 | $-CH(CH_3)-$ | 2′ | NH | – |
| 1 f | H | 4 | $-CH(CH_3)-$ | 2′ | NH | HCl |
| 2 b | H | 4 | $-CH_2-$ | 2′ | NH | – |
| 2 c | H | 4 | $-CH_2-$ | 2′ | NH | HCl |
| 2 d | H | 4 | $-CH_2-$ | 2′ | NH | HCl · $H_2O$ |
| 3 c | H | 4 | $-CH_2-$ | 3′ | NH | – |
| 4 c | H | 4 | $-C(=O)-$ | 2′ | NH | – |
| 5 b | H | 4 | $-CH_2-$ | 2′ | NEt | – |
| 6 b | Et | 5 | $-CH_2-$ | 2′ | NH | – |
| 7 b | Me | 5 | $-C(CH_3)(OH)-$ | 2′ | $CH_2$ | – |
| 7 c1 | Me | 5 | $-C(=CH_2)-$ | 2′ | $CH_2$ | – |

Table 2 (contd.)

<table>
<tr><th>Example</th><th>R[1]</th><th>Bonding position a</th><th>A</th><th>Bonding position b</th><th>X</th><th>Salt, etc.</th></tr>
<tr><td>7c2</td><td>Me</td><td>5</td><td>$CH_3$<br>|<br>$-CH-$</td><td>2'</td><td>$CH_2$</td><td>−</td></tr>
<tr><td>8 b</td><td>Me</td><td>5</td><td>OH<br>|<br>$-CH-$</td><td>2'</td><td>$CH_2$</td><td>−</td></tr>
<tr><td>8 c</td><td>Me</td><td>5</td><td>$-CH_2-$</td><td>2'</td><td>$CH_2$</td><td>−</td></tr>
<tr><td>9 b</td><td>Et</td><td>5</td><td>OH<br>|<br>$-CH-$</td><td>2'</td><td>$CH_2$</td><td>−</td></tr>
<tr><td>9 c</td><td>Et</td><td>5</td><td>$-CH_2-$</td><td>2'</td><td>$CH_2$</td><td>−</td></tr>
<tr><td>10 b</td><td>Et</td><td>5</td><td>$CH_3$<br>|<br>$-C-$<br>|<br>OH</td><td>2'</td><td>$CH_2$</td><td>−</td></tr>
<tr><td>10 c</td><td>Et</td><td>5</td><td>$CH_2$<br>‖<br>$-C-$</td><td>2'</td><td>$CH_2$</td><td>−</td></tr>
<tr><td>10 d</td><td>Et</td><td>5</td><td>$CH_3$<br>|<br>$-CH-$</td><td>2'</td><td>$CH_2$</td><td>−</td></tr>
<tr><td>11 e</td><td>Me</td><td>5</td><td>$-CH_2-$</td><td>2'</td><td>NH</td><td>−</td></tr>
<tr><td>12 e</td><td>H</td><td>4</td><td>$-CH_2CH_2-$</td><td>2'</td><td>NH</td><td>−</td></tr>
<tr><td>13</td><td>n−Pr</td><td>5</td><td>$-CH_2-$</td><td>2'</td><td>NH</td><td></td></tr>
</table>

Table 2 (contd.)

| Example | $R^1$ | Bonding position a | A | Bonding position b | X | Salt, etc. |
|---|---|---|---|---|---|---|
| 14c | iso-Pr | 5 | $-CH_2-$ | 2' | NH | – |
| 15c | 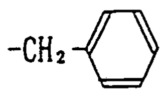 | 5 | $-CH_2-$ | 2' | NH | – |
| 16c | H | 4 | 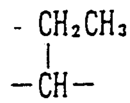 | 2' | NH | – |
| 17c | H | 4 | 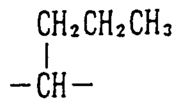 | 2' | NH | – |
| 18c | H | 4 | 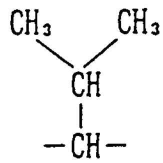 | 2' | NH | – |
| 19c | H | 4 | 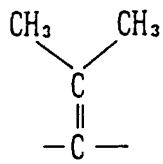 | 2' | NH | HCl |
| 20c | H | 4 | 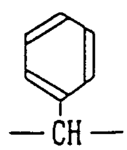 | 2' | NH | HCl |
| 21e | H | 4 | 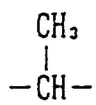 | 3' | NH | – |
| 22e | Me | 5 | 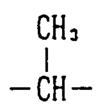 | 2' | NH | – |
| 23d | H | 4 | 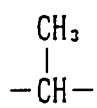 | 2' | NMe | – |

Table 2 (contd.)

| Example | $R^1$ | Bonding position a | A | Bonding position b | X | Salt, etc. |
|---|---|---|---|---|---|---|
| 24d | $-CH_2-$ 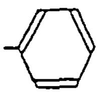 | 5 | 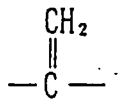 | 2' | NH | HCl |
| 25 | H | 4 | $-CH_2-$ | 2' | $CH_2$ | – |

Other compounds shown in Table 3 can be synthesized making use of the production methods of the compounds of the present invention described in the instant specification, the production methods of the Examples, other methods well known to those skilled in the art and modified methods thereof, without requiring special experiments.

Table 3

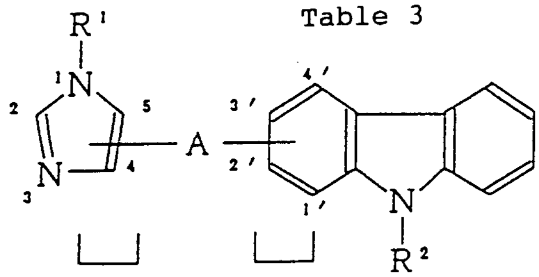

Bonding position a    Bonding position b

(I)

| Compound No. | $R^1$ | Bonding position a | A | Bonding position b | $R^2$ |
|---|---|---|---|---|---|
| 1 | H | 4 | 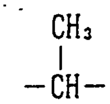 | 2′ | Et |
| 2 | H | 4 | 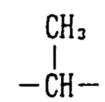 | 2′ | n-Pr |
| 3 | H | 4 | 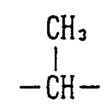 | 2′ | n-Bu |
| 4 | H | 4 | 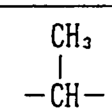 | 2′ | iso-Pr |
| 5 | Et | 5 | 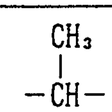 | 2′ | H |
| 6 | n-Pr | 5 | 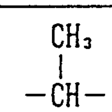 | 2′ | H |
| 7 | n-Bu | 5 | 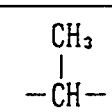 | 2′ | H |
| 8 | iso-Pr | 5 | 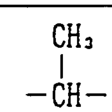 | 2′ | H |
| 9 | H | 4 | 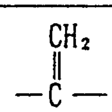 | 2′ | H |
| 10 | H | 4 | 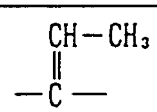 | 2′ | H |

Table 3 (contd.)

| Compound No. | $R^1$ | Bonding position a | A | Bonding position b | $R^2$ |
|---|---|---|---|---|---|
| 11 | H | 4 | $CH_2CH_2CH_2CH_3$<br>\|<br>$-CH-$ | 2' | H |
| 12 | H | 4 | $CH_2CH_3$<br>\|<br>$-CH-$ | 3' | H |
| 13 | H | 4 | $CH_2CH_2CH_3$<br>\|<br>$-CH-$ | 3' | H |
| 14 | H | 4 | $CH_2CH_2CH_2CH_3$<br>\|<br>$-CH-$ | 3' | H |
| 15 | H | 4 | $CH_3$ $CH_3$<br>CH<br>\|<br>$-CH-$ | 3' | H |
| 16 | H | 4 | $CH_3$<br>\|<br>$-CH-$ | 3' | Me |
| 17 | H | 4 | $CH_3$<br>\|<br>$-CH-$ | 3' | Et |
| 18 | H | 4 | $CH_3$<br>\|<br>$-CH-$ | 3' | n-Pr |
| 19 | H | 4 | $CH_3$<br>\|<br>$-CH-$ | 3' | n-Bu |
| 20 | H | 4 | $CH_3$<br>\|<br>$-CH-$ | 3' | iso-Pr |
| 21 | Me | 5 | $CH_3$<br>\|<br>$-CH-$ | 3' | H |
| 22 | Et | 5 | $CH_3$<br>\|<br>$-CH-$ | 3' | H |

Table 3 (contd.)

| Compound No. | $R^1$ | Bonding position a | A | Bonding position b | $R^2$ |
|---|---|---|---|---|---|
| 23 | n-Pr | 5 | 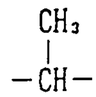 | 3' | H |
| 24 | n-Bu | 5 | 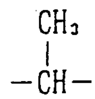 | 3' | H |
| 25 | iso-Pr | 5 | 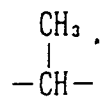 | 3' | H |
| 26 | H | 4 | 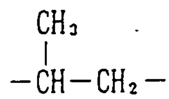 | 2' | H |
| 27 | H | 4 | 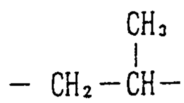 | 2' | H |
| 28 | H | 4 | $-CH_2-$ | 4' | H |
| 29 | H | 4 | $-CH_2-$ | 1' | H |
| 30 | H | 4 | 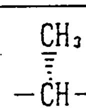 | 2' | H |
| 31 | H | 4 | 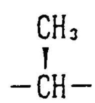 | 2' | H |
| 32 | H | 4 | 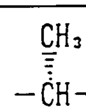 | 3' | H |
| 33 | H | 4 | 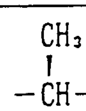 | 3' | H |
| 34 | H | 4 | 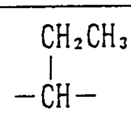 | 2' | Me |

Table 3 (contd.)

| Compound No. | $R^1$ | Bonding position a | A | Bonding position b | $R^2$ |
|---|---|---|---|---|---|
| 35 | H | 4 | $CH_2CH_3$<br>\|<br>$-CH-$ | 2′ | Et |
| 36 | Me | 5 | $-CH_2-$ | 2′ | Me |
| 37 | Me | 5 | $-CH_2-$ | 2′ | Et |
| 38 | Me | 5 | $CH_3$<br>\|<br>$-CH-$ | 2′ | Me |
| 39 | Me | 5 | $CH_3$<br>\|<br>$-CH-$ | 2′ | Et |
| 40 | Me | 5 | $CH_2CH_3$<br>\|<br>$-CH-$ | 2′ | H |
| 41 | Me | 5 | $CH_2CH_2$<br>\|<br>$-CH-$ | 2′ | Me |
| 42 | Me | 5 | $CH_2CH_3$<br>\|<br>$-CH-$ | 2′ | Et |
| 43 | Et | 5 | $-CH_2-$ | 2′ | Me |
| 44 | Et | 5 | $-CH_2-$ | 2′ | Et |
| 45 | Et | 5 | $CH_3$<br>\|<br>$-CH-$ | 2′ | Me |
| 46 | Et | 5 | $CH_3$<br>\|<br>$-CH-$ | 2′ | Et |

Table 3 (contd.)

| Compound No. | $R^1$ | Bonding position a | A | Bonding position b | $R^2$ |
|---|---|---|---|---|---|
| 47 | Et | 5 | 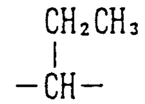 | 2' | H |
| 48 | Et | 5 | 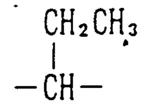 | 2' | Me |
| 49 | Et | 5 | 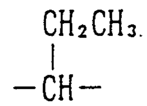 | 2' | Et |

Formulation example of oral preparation

| Composition | |
|---|---|
| Tablet materials | |
| Compound of the present invention | 1.0 mg |
| Lactose | 76.4 mg |
| Corn starch | 19.3 mg |
| Hydroxypropylcellulose | 3.0 mg |
| Magnesium stearate | 0.3 mg |
| Sub total | 100 mg |
| Coat materials | |
| Hydroxypropylmethylcellulose 2910 | 2.9 mg |
| Polyethylene glycol 6000 | 0.4 mg |
| Titanium oxide | 1.6 mg |
| Talc | 0.1 mg |
| Sub total | 5 mg |
| Total | 105 mg |

1 mg tablets

The compound of the present invention (7 g) and 534.8 g of lactose were mixed in a polyethylene bag. This mixture was pulverized using Sample Mill (manufactured by Hosokawa Micron). The pulverized mixture (541.8 g) was uniformly mixed with 135.1 g of corn starch in a fluidized granulation coating apparatus (manufactured by Ohkawara Seisakusho). This was made into granules by spraying 210 g of 10% hydroxypropylcellulose solution. After drying, the granules were passed through a 20 mesh screen, mixed with 2.1 g of magnesium stearate and then made into tablets of 100 mg per tablet using a die/punch system of ∅ 6.5 mm × 7.8 R by a rotary tablet making machine (manufactured by Hata Tekko-sho). Using a coating machine (manufactured by Freund Sangyo), 350 g of a coating solution containing 20.3 g of hydroxypropylmethylcellulose, 2.8 g of polyethylene glycol 6000, 11.2 g of titanium oxide and 0.7 g of talc was sprayed to the thus prepared tablets, thereby obtaining film coated tablets in which each tablet was coated with 5 mg of the coat materials.

## Claims

1. An imidazole derivative represented by the following general formula (I), a salt thereof, a hydrate thereof or a solvate thereof

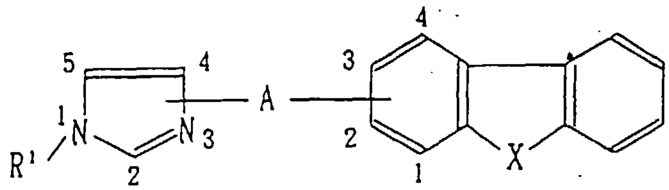

(I)

(symbols in the formula have the following meanings;

A: a lower alkylene group unsubstituted or substituted with a hydroxyl group, an aryl group, a lower alkylidene group or an oxo group (=O),
X: a methylene group or a group represented by a formula $-NR^2-$,
$R^1$: a hydrogen atom, a lower alkyl group or an aralkyl group, and
$R^2$: a hydrogen atom or a lower alkyl group).

2. A compound, a salt thereof, a hydrate thereof or a solvate thereof according to claim 1, wherein it is characterized in that A is linked at the 4-position or 5-position of the imidazole ring.

3. A compound, a salt thereof, a hydrate thereof or a solvate thereof according to claim 2, wherein it is characterized in that A is linked at the 2- or 3-position of the ring

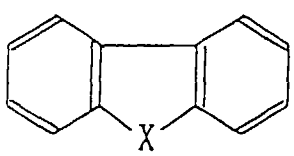

4. A compound, a salt thereof, a hydrate thereof or a solvate thereof according to claim 3, wherein A is a lower alkylene group unsubstituted or substituted with an aryl group or a lower alkylidene group.

5. A compound, a salt thereof, a hydrate thereof or a solvate thereof according to claim 4, wherein A is a lower alkylene group unsubstituted or substituted with a lower alkylidene group.

6. A compound, a salt thereof, a hydrate thereof or a solvate thereof according to claim 5, wherein X is a group represented by a formula $NR^2$.

7. 2-[1-(1H-Imidazol-4-yl)ethyl]-9H-carbazole, a salt thereof, a hydrate thereof or a solvate thereof.

8. 3-[1-(1H-Imidazol-4-yl)ethyl]-9H-carbazole, a salt thereof, a hydrate thereof or a solvate thereof.

9. 2-(1H-Imidazol-4-ylmethyl)-9H-carbazole, a salt thereof, a hydrate thereof or a solvate thereof.

**10.** 3-(1H-Imidazol-4-ylmethyl)-9H-carbazole, a salt thereof, a hydrate thereof or a solvate thereof.

**11.** A pharmaceutical composition which comprises the compound of claim 1 or a pharmaceutically acceptable salt thereof as its active ingredient.

**12.** A steroid 17-20 lyase inhibitor which comprises the compound of claim 1 or a pharmaceutically acceptable salt thereof as its active ingredient.

**13.** The steroid 17-20 lyase inhibitor according to claim 12, which is an agent for the prevention or treatment of diseases caused by androgen and/or estrogen.

**14.** The steroid 17-20 lyase inhibitor according to claim 13, which is an agent for the prevention or treatment of prostate cancer, benign prostatic hyperplasia, virilism, hirsutism, breast cancer, mastopathy, hysteromyoma and endometriosis.

## INTERNATIONAL SEARCH REPORT

International application No.
PCT/JP96/00490

**A. CLASSIFICATION OF SUBJECT MATTER**

Int. $Cl^6$ C07D233/58, 403/06, A61K31/415

According to International Patent Classification (IPC) or to both national classification and IPC

**B. FIELDS SEARCHED**

Minimum documentation searched (classification system followed by classification symbols)

Int. $Cl^6$ C07D233/58, 403/06, A61K31/415

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| Y | JP, 60-136569, A (Eli Lilly and Co.), July 20, 1985 (20. 07. 85) & EP, 138415, A & US, 4533670, A | 1 - 5 |
| A | JP, 57-53466, A (Daiichi Seiyaku Co., Ltd.), March 30, 1982 (30. 03. 82)(Family: none) | 1 - 5 |

☐ Further documents are listed in the continuation of Box C. ☐ See patent family annex.

* Special categories of cited documents:
- "A" document defining the general state of the art which is not considered to be of particular relevance
- "E" earlier document but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| April 30, 1996 (30. 04. 96) | May 14, 1996 (14. 05. 96) |
| Name and mailing address of the ISA/ Japanese Patent Office | Authorized officer |
| Facsimile No. | Telephone No. |

Form PCT/ISA/210 (second sheet) (July 1992)